# EUROPEAN PATENT APPLICATION

(11) **EP 3 015 855 A1**
(43) Date of publication of application: **04.05.2016**
(21) Application number: 14190482.1
(22) Date of filing: 27.10.2014
(51) Int. Cl.: G01N 24/08, A61B 5/055, A61K 49/06, G01R 33/28, G01R 33/465, G01R 33/56

(54) **Metal biosensors based on compounds with metal-sensitive chemical shifts for magnetic resonance spectroscopy and imaging**

(71) Applicant: Klinikum rechts der Isar der Technischen Universität München, 81675 München (DE); Helmholtz Zentrum München Deutsches Forschungszentrum für Gesundheit und Umwelt GmbH, 85764 Neuherberg (DE)
(72) Inventor: Westmeyer, Gil Gregor, 80804 Munich (DE); Mishra, Anurag, 80804 Munich (DE); Pariani, Giorgio, 81369 München (DE)
(74) Representative: Engelhard, Markus

(57) **Abstract**

The present invention relates to the use of compounds with at least one metal-sensitive chemical shift for determining metal concentrations and/or measuring metal concentration changes, wherein the compound is selected from the group of: pyro-EGTA, EGTA, EDTA, AATA, APTRA, BAPTA, HIDA, citrate, CarboxyGlutamate (CGlu), arylazo chromotopic acid, beta-diketone (crown-ether), mono-, di-, or tri-pyridyl aniline, mono-, di-, or tri-pyridyl amine, trimethylphenylammonium, or derivatives thereof. The present invention further relates to biosensors comprising at least one of the compounds. The present invention also relates to the use of the compounds for diagnosing and/or monitoring treatment of a disease causing changes in metal concentrations. The present invention is furthermore related *to in vitro* and *in vivo* methods for determining metal concentration and/or measuring metal concentration changes using the compounds or biosensors. The present invention also relates to methods of diagnosing and/or monitoring treatment of a disease causing changes in metal concentrations wherein the compounds or biosensors are applied. The present invention also relates to use of the compounds or biosensors in quality control of food or in the examination of plants and organisms or for monitoring of environmental resources. The present invention further relates to novel derivatives of pyro-EGTA, AATA, APTRA and BAPTA as well as their uses.

## Description

The present invention relates to the use of compounds with at least one metal-sensitive chemical shift for determining metal concentrations and/or measuring metal concentration changes, wherein the compound is selected from the group of: pyro-EGTA, EGTA, EDTA, AATA, APTRA, BAPTA, HIDA, citrate, CarboxyGlutamate (CGlu), arylazo chromotopic acid, beta-diketone (crown-ether), mono-, di-, or tri-pyridyl aniline, mono-, di-, or tri-pyridyl amine, trimethylphenylammonium, or derivatives thereof. The present invention further relates to biosensors comprising at least one of the compounds. The present invention also relates to the use of the compounds for diagnosing and/or monitoring treatment of a disease causing changes in metal concentrations. The present invention is furthermore related to *in vitro* and *in vivo* methods for determining metal concentration and/or measuring metal concentration changes using the compounds or biosensors. The present invention also relates to methods of diagnosing and/or monitoring treatment of a disease causing changes in metal concentrations wherein the compounds or biosensors are applied. The present invention also relates to use of the compounds or biosensors in quality control of food or in the examination of plants and organisms or for monitoring of environmental resources. The present invention further relates to novel derivatives of pyro-EGTA, AATA, APTRA and BAPTA as well as their uses.

### BACKGROUND OF THE INVENTION

Metal ions are of fundamental importance for biological processes since they function as essential constituents of structural proteins and enzymes, or as signaling molecules and second messengers. The most prominent example of the last category is calcium, which is important in many signal-transduction cascades and also essential for the signaling in electrically excitable cells, in particular the electrochemical conversion at the presynaptic synapses of neurons mediated through voltage gated influx and intracellular release of calcium as a function of membrane potential. Organ(ism)s thus have to tightly control the concentration of metals and their spatiotemporal distribution as many factors including e.g. increased metabolic demand, malnutrition or contact with toxic concentrations of metals present for instance in environmental pollutants can cause deflections from the physiological range.

Synthetic metal sensors are available for optical readouts based on absorbance (Durham *et al.,* 1983) and fluorescence (Carter *et al.,* 2014). There also exist genetically encoded sensors for readout via fluorescence (Looger *et al.,* 2013) and bioluminescence (in conjunction with appropriate synthetic substrates). These photon-dependent metal sensors however all suffer from the poor penetration of photons in biological tissue or in opaque and/or turbid samples. The penetration depth depends on the specific method and desired resolution; even optimal use of near-infrared illumination and independence scattering as achieved by opt acoustics however still limits the maximum penetration depth in biological tissues to a few centimeters (Ntziachristos *et al.,* 2010). For these photon-dependent imaging methods to measure deeper structures *in vivo,* invasive methods are necessary that e.g. surgically insert optical guides such as fiber bundles into tissue. For diagnostic measurements of *in vitro* or *ex-vivo* samples (such as bodily fluids and tissue as well also non-medical samples such as waste water) photon-dependent methods are also severely limited in their performance by the samples' opacity and turbidity.

It would therefore be of great value for biomedical diagnostics as well as for applications in food and environmental testing if robust photon-independent detection of metals in opaque and turbid media and non-invasive *in vivo* imaging of their distribution in organ(ism)s could be achieved at sub-millimeter resolution.

One non-invasive photon-independent method that fulfills these criteria is nuclear Magnetic Resonance Imaging (MRI). For this method that routinely achieves whole-body coverage in humans, metal-sensitive contrast agents have been developed previously. They fall into four categories according to their physical mechanism: T₂ relaxation agents, T₁ relaxation agents, CEST agents and chemical shift agents.

A first category of metal-sensitive contrast agents consists of (super)paramagnetic nanoparticles in the case that they consist of iron-oxide abbreviated SPIOs, which mainly give rise to so-called T₂ or T₂* contrast in appropriate MRI sequences. A change in the agglomeration state of SPIOS alters their T₂/T₂* contrast (Perez *et al.,* 2002), an effect that can be made specific to analytes of interest via surface modifications with appropriate affinities for the analytes. Using peptide and protein surface modifications, this effect was used to generate SPIOs that exhibited calcium-dependent agglomeration and accompanying T₂ signal changes (Atanasijevic *et al.,* 2006). T₂ sensors can give contrast at nanomolar concentrations (Shapiro *et al.,* 2006) and metal-dependent contrast changes can be a few fold. Due to their large size though, these agents are very difficult to deliver to tissues. In addition, their response time is only on the order of a few seconds and a function of their concentration due to the complicated agglomeration process (Shapiro *et al.,* 2006). In the case of iron-oxide particles, there are data which show that after degradation of the nanostructure, the iron is metabolized by the organism, one of the reasons why the U.S. Food and Drug Administration has approved some SPIOs for imaging of humans under certain conditions. However, there remain concerns about the biosafety of SPIOs such that a potential use for *e.g.* neuroimaging is off-label (Winer *et al.,* 2012). Whereas the agglomeration-based T₂ contrast mechanism using SPIOs agent is used for *in* vitro-diagnostics (*e.g.* by the company T2 Biosystems), successful *in vivo* use has not been demonstrated probably due to the difficulties in preventing unspecific agglomeration events and control delivery and stoichiometry in the target tissue.

A second category of sensors generates metal-dependent MRI contrast changes by altering the so-called T₁ spin-lattice through modulating access of protons to a macrocycle-coordinated metal (in most cases a lanthanide) as a function of metal binding. With respect to analytes, calcium, magnesium, zinc, iron and potassium have been targeted, but the maximum relaxivity changes of any of these relaxation agents was only a factor of two to three with most of these contrast agents exhibiting much smaller signal changes (Que *et al.,* 2010). Toxic metals such as cadmium and nickel or lead have not been targeted with NMR-based metal sensors so far. Because of the low sensitivity of proton MRI and the low relaxivity of lanthanide-based MRI contrast agents, these contrast agents have to be used at concentrations of hundreds of micromolar (Caravan *et al.,* 2006) and even higher concentrations for chemical shift agents that rely on the natural abundance of heteronuclei. This low signal can only be compensated for by long examination times and spatial averaging compromising spatial and temporal resolution to a degree that is not practical for imaging application in preclinical animal models or small specimens. For clinical applications in human subjects, long radiofrequency irradiation is also limited by the allowed specific absorption rate (SAR). Furthermore, a concern for the (pre)clinical use of lanthanoid-complexes is their potential toxicity, which may cause severe syndromes such as nephrogenic systemic fibrosis.

A third category of metal-sensitive MRI contrast agents are not based on relaxation rate changes but rely on the detection of chemical shift, which are changes of the nuclear spin frequency due to changes (relative to some standard such as tetramethylsilane (TMS)) of the microenvironment that the respective nucleus is affected by. ¹⁹F containing moieties based on the calcium chelators BAPTA and Quin2 have been generated (Smith *et al.,* 1983; Steenbergen *et al.,* 1987; Schanne *et al.,* 1990) ; in particular, the 5,5'-difluoro derivative (5F-BAPTA) exhibits robust enough chemical shifts. In Robitaille *et al.* (1992) the divalent metal chelator AATA is described that was modified with ¹³C and a chemical shift was measured via Proton-observed carbon-edited (POCE) NMR spectroscopy. However, because of the lower concentration of these nuclei compared with protons, their detection via NMR is inherently less sensitive.

A fourth category of MRI metal sensors are based on so-called chemical exchange saturation transfer (CEST), *i.e.* the selective saturation of the magnetization of a pool of nuclei (*e.g.* protons) associated with the CEST contrast agent and observation of the signal change due to chemical exchange with that pool. The fluorinated BAPTA derivative 5F-BAPTA was used in CEST mode to enhance the sensitivity to detect calcium (Bar-Shir *et al.*) and was recently extended with a molecule that was also fluorinated at the 4 position (5,5', 6,6'-tetrafluoro-BAPTA) that can also detect zinc and iron (Bar-Shir *et al.,* 2014). Angelovski *et al.* (2011) have developed an agent for calcium in which a paramagnetic gadolinium enhances the CEST effect (PARACEST). Both of these methods however require micromolar concentrations of the CEST agent and the temporal resolution of the CEST method is generally slower than for relaxation agents due to the duration of saturation pulses.

In general, the above-mentioned methods all suffer from a lack of sensitivity, obviously one of the major obstacles for *in vivo* MRI in general, necessitating the use of higher micromolar if not millimolar concentrations of the contrast agent.
To improve MRI's sensitivity, several so-called hyperpolarization methods have been developed that can increase the polarization of nuclear spins and thus the available signal (Viale *et al.*). In the case of the noble gases helium (³He) and xenon (¹²⁹Xe), hyperpolarization can be achieved via optical pumping. In case the contrast agent of interest can be generated through hydrogenation reaction, Para-Hydrogen Induced Polarization (PHIP) can be employed.

Another fairly general method for hyperpolarization is dissolution Dynamic Nuclear Polarization (DNP) based on the transfer of spin polarization from electrons to nuclei by radiofrequency irradiation of the compound together with a radical at low temperatures with radiofrequency near the electron resonance frequency. The resultant increase in signal of up to several orders of magnitude allows the measurement of insensitive nuclei such as ¹³C (Ardenkjaer-Larsen *et al.,* 2003). These nuclei may be contained in hyperpolarizable small contrast agent compounds, which may be indistinguishable from endogenous molecules by the biochemical processes of interest and are thus also probably non-toxic. A prominent example of this approach is hyperpolarized [1-¹³C]-pyruvate that can be used to map the distribution of the enzyme LDH via detecting the chemical shifts occurring upon conversion of pyruvate to lactate. This can for instance be useful for the spatiotemporal detection of tumor cells that may exhibit pathological LDH activity as may be the case for prostate carcinoma (Nelson *et al.,* 2013 -1 and Nelson *et al.,* 2013 -2). A DNP-hyperpolarized molecule based on ⁸⁹Y has been described that shows a pH-dependent chemical shift (Jindal *et al.,* 2010).

Nonaka *et al.* (2013) have recently published a ¹⁵N-containing compound, namely [¹⁵N, D₉]trimethylphenylammonium ([¹⁵N, D₉]TMPA) that can be hyperpolarized by DNP and exhibits a chemical shift of the ¹⁵N moiety when coupled to a metal chelator APTRA. However, the chemical shift is not metal-specific.

In summary, there is a need in the art of improved means and methods for measuring metal concentration changes, preferably with spatial resolution and in real time in a photon-independent fashion *in vitro, ex vivo* and especially non-invasively *in vivo.* For the latter, a method that can simultaneously and specifically and robustly detect several metals at an increased sensitivity is needed.

### SUMMARY OF THE INVENTION

According to the present invention this object is solved by the use of a compound with at least one metal-sensitive chemical shift for determining metal concentrations and/or measuring metal concentration changes, wherein the compound is selected from the group of:
pyro-EGTA,
EGTA,
EDTA,
AATA,
APTRA,
BAPTA,
HIDA,
citrate,
carboxyglutamate (CGlu),
or derivatives thereof,
arylazo chromotopic acid derivatives,
beta-diketone (crown-ether) derivatives,
mono-, di-, or tri-pyridyl aniline derivatives,
mono-, di-, or tri-pyridyl amine derivatives,
trimethylphenylammonium derivatives.
and their hydrates, salts, solutions, stereoisomers.

According to the present invention this object is solved by a biosensor for determining metal concentrations and/or measuring metal concentration changes, comprising
(i) at least one compound with at least one metal-sensitive chemical shift of the present invention,
(ii) optionally, a reference compound,
(iii) optionally, one or several pharmaceutically acceptable carriers and/or excipients.

According to the present invention this object is solved by providing the compound of the present invention or the biosensor of the present invention for use in *in vivo* magnetic resonance imaging (MRI), magnetic resonance spectroscopy (MRS) or magnetic resonance tomography (MRT) and/or absorbance/transmission, reflection, fluorescence or optoacoustic measurements and imaging.

According to the present invention this object is solved by providing the compound of the present invention or the biosensor of the present invention for use in diagnosing and/or monitoring treatment of diseases causing changes in metal concentrations,

According to the present invention this object is solved by the use of the compound of the present invention as metal sensor for *in vitro* or *ex-vivo* NMR spectroscopy.

According to the present invention this object is solved by an *in vitro* method for determining metal concentration and/or measuring metal concentration changes, comprising the steps of
(i) providing a sample,
(ii) adding a compound with at least one metal-sensitive chemical of the present invention or the biosensor of the present invention to the sample,
(iii) performing magnetic resonance imaging (MRI) or magnetic resonance spectroscopy (MRS) and thereby determining the spatial distribution of metal concentration or metal concentration changes of or in the sample
   (1) by obtaining a chemical shift difference between at least one metal-sensitive chemical shift of the compound and a metal-independent chemical shift, such metal-independent chemical shift acting as a reference chemical shift, or
   (2) by measurement of the absolute chemical shift, or
   (3) by measuring chemical shift differences involving at least one metal-sensitive shift, or
   (4) by obtaining additional measurements of metal-insensitive signals that relate to the concentration of the sensor, for instance based on radioactivity, absorbance, fluorescence or optoacoustic signal from the metal-sensor containing the appropriate radioactive moiety, fluorophore/chromophore.

According to the present invention this object is solved by an *in vivo* method for determining metal and/or measuring metal concentration changes, preferably in real-time, comprising the steps of
(i) applying or administering a compound with at least one metal-sensitive chemical shift of the present invention or a biosensor of the present invention to the body of a patient or non-human animal,
(ii) performing magnetic resonance imaging (MRI) and thereby determining one or several metal concentrations or metal concentration changes of or in the body of said patient or non-human animal
   (1) by correcting a spatiotemporally resolved metal-sensitive signal for differences in local and time-dependent signal intensities, *e.g.* due to differences in the local concentration of the metal sensors, and
      by obtaining the chemical shift and/or its integrated peak area and/or peak amplitude from at least one metal-sensitive chemical shift and a metal-insensitive reference in the form chemical shift and/or integrated peak area from at least one metal-insensitive chemical shift or a function of metal-sensitive shifts,
      or
   (2) by measurement of the absolute chemical shift, integrated peak area or peak amplitude,
      or
   (3) by measuring a metal-insensitive MRI signal from another nucleus such as proton via a lanthanide, ¹H and/or ¹³C and/or ¹⁵N and/or ¹⁹F and/or ²⁹Si and/or ³¹P and/or ⁸⁹Y, or
   (4) by combination with a PET measurement *e.g.* via ¹⁸F or fluorescence or optoacoustics *e.g.* via a chromophore or fluorophore,

According to the present invention this object is solved by a method of diagnosing and/or monitoring treatment of a disease causing changes in metal concentrations, comprising the steps of
(i) applying or administering a compound with at least one metal-sensitive chemical shift of the present invention or a biosensor of the present invention to the body of a patient or non-human animal,
(ii) performing magnetic resonance imaging (MRI) or magnetic resonance spectroscopy (MRS) and thereby determining one or several metal concentrations or metal concentration changes of or in the body of said patient or non-human animal
   (1) by obtaining over time the chemical shift and/or its integrated peak area and/or its peak amplitude from at least one metal-sensitive chemical shift and a metal-insensitive reference in the form of a chemical shift and/or its integrated peak area and/or peak amplitude from at least one metal-insensitive chemical shift or a function of metal-sensitive shifts,
      or
   (2) by measurement of the absolute chemical shift, integrated peak area or peak amplitude,
      or
   (3) by measuring a metal-insensitive MRI signal from another nucleus such as proton via a lanthanide, ¹H and/or ¹³C and/or ¹⁵N and/or ¹⁹F and/or ²⁹Si and/or ³¹P and/or ⁸⁹Y, or
   (4) by combination with a PET measurement *e.g.* via ¹⁸F or fluorescence or optoacoustics *e.g.* via a chromophore or fluorophore, or CT via a radiocontrast agent,
(iii) calculating metal concentration maps based on spatially resolved metal concentration values or metal concentration changes determined in step (ii).

According to the present invention this object is solved by the use of the compound of the present invention or the biosensor of the present invention in quality control of food or in the examination of plants and organisms, or for monitoring of environmental resources with respect to pollution and harmful metal concentrations.

According to the present invention this object is solved by a derivative or analog of pyro-EGTA, AATA, APTRA or BAPTA, comprising metal affinity modifying moiety/moieties, wherein the metal affinity modifying moiety/moieties is/are selected from halogen (*e.g.* Cl, Br, I), a hyperpolarizable nucleus (e.g. ¹⁹F, or ³¹P, ²⁹Si, ¹⁵N) or NH₂.

According to the present invention this object is solved by using the derivative or analog of pyro-EGTA, AATA, APTRA or BAPTA of the present invention for the uses and in the methods according to the present invention

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

### Metal biosensors based on metal chelators with metal-sensitive chemical shift(s)

As discussed above, the present invention provides the use of a compound with at least one metal-sensitive chemical shift for determining metal concentrations and/or measuring metal concentration changes.

Current NMR-based metal sensors fail to solve the objectives of the present invention due to a combination of their low Signal-to-Noise Ratio (SNR) and limitation by SAR, potential toxicity and difficulties of delivery to the tissue of interest. Current NMR-based metal sensors have thus not emerged as robust tools for *in vitro, ex vivo* or *in vivo* use in preclinical animal models and it is unlikely that these methods will translate into the clinic or biomedical research for use in humans.

The inventors have now surprisingly found that it is possible to make use of the metal-dependent displacement of chemical shifts in compounds for determining one or several metal concentrations and/or measuring metal concentration changes. In particular, this concerns the displacement of
metal-sensitive ¹H chemical shifts in ¹H-magnetic resonance imaging and/or ¹H magnetic resonance spectroscopy, and/or
metal-sensitive ¹³C chemical shifts in ¹³C-magnetic resonance imaging and/or ¹³C magnetic resonance spectroscopy, and/or
metal-sensitive ¹⁵N chemical shifts in ¹⁵N-magnetic resonance imaging and/or ¹⁵N magnetic resonance spectroscopy, and/or
metal-sensitive ¹⁹F chemical shifts in ¹⁹F-magnetic resonance imaging and/or ¹⁹F magnetic resonance spectroscopy, and/or
metal-sensitive ²⁹Si chemical shifts in ²⁹Si-magnetic resonance imaging and/or ²⁹Si magnetic resonance spectroscopy, and/or
metal-sensitive ³¹P chemical shifts in ³¹P-magnetic resonance imaging and/or ³¹P magnetic resonance spectroscopy, and/or
metal-sensitive ⁸⁹Y chemical shifts in ⁸⁹Y-magnetic resonance imaging and/or ⁸⁹Y magnetic resonance spectroscopy.

Preferably, a compound with at least one metal-sensitive chemical shift is ¹H-labelled, ¹³C-labelled, ¹⁵N-labelled, ¹⁹F-labelled, ²⁹Si-labelled, ³¹P-labelled, and/or ⁸⁹Y-labelled.

As used herein "chemical shift" refers to a small change in the spin frequency of a nucleus due to its microenvironment with respect to a reference frequency of a suitable standard, such as tetramethylsilane (TMS). Chemical shift is commonly expressed in parts-per-million (ppm).

The term "displacement of a chemical shift", as used herein is meant to refer to a change in position of the respective chemical shift. In this context, "displacement of a chemical shift" is preferably meant to refer to a change in position of a ¹H, ¹³C, ¹⁵N, ¹⁹F, ²⁹Si, ³¹P and ⁸⁹Y chemical shift. The measure of metal-concentration can derive from the displacement of a chemical shift and/or from the integrated area of a displaced chemical shift and/or from peak amplitude of a displaced chemical shift.

Preferably, a compound with at least one metal-sensitive chemical shift comprises one or more metal-sensitive chemical shifts (namely one or more metal-sensitive ¹H and/or ¹³C and/or ¹⁵N and/or ¹⁹F and/or ²⁹Si and/or ³¹P and/or ⁸⁹Y chemical shifts), such as two, three, four or more.

Here, a novel series of metal-biosensors is presented (based on several classes of metal chelators, such as pyro-EGTA, EGTA, EDTA, AATA, APTRA, BAPTA, HIDA, citrate, carboxyglutamate (CGlu), arylazo chromotopic acid, beta-diketone (crown-ether), mono/di/tri-pyridyl aniline or mono/di/tri-pyridyl amine, trimethylphenylammonium, incorporating one or more hyperpolarizable isotopes) for magnetic resonance measurements that are sensitive and specific to various metals in concentration ranges relevant for detection of deviations from the physiological range.

As used herein "magnetic resonance" refers to the observation of Larmor precession in a magnetic field (see Ernst, 1997 and de Graaf, 2007), and includes measurements at a NMR spectrometer, an NMR microimaging system, an MRI scanner, a low-field NMR device, microfluidic arrays ("NMR on a chip"), and/or combinations thereof. Measurement includes all variations of spatially and/or spectrally resolved magnetic resonance techniques, such as magnetic resonance spectroscopy (MRS), magnetic resonance imaging (MRI), or magnetic resonance spectroscopic imaging (MRSI).

Preferably, the compounds of the present invention comprise at least one carboxylic group, amide group, nitrogen, or oxygen group (preferably one or more of these groups, such as two, three, four or more) such that the specific metal of interest is effectively coordinated.

According to the present invention, said compound with at least one metal-sensitive chemical shift exhibits at least one NMR resonance with a metal-sensitive chemical shift in an NMR spectrum.

According to the present invention, the compound is preferably based on several classes of metal chelators and is more preferably selected from the group of:

| | (IUPAC) name |
|---|---|
| pyro-EGTA | 2,2',2'',2'''-(2,2'-(1,2-phenylene bis(oxy))bis(ethane-2,1-diyl)) bis(azanetriyl)tetraacetic acid |
| EGTA | ethylene glycol-bis(2-aminoethylether)-N,N,N',N'-tetraacetic acid |
| EDTA | 2,2',2'',2'''-(ethane-1,2-diyldinitrilo)tetraacetic acid (ethylenediamine tetraacetic acid) |
| AATA | 2,2'-(2-(2-(2-(bis(carboxymethyl)amino)ethoxy)ethoxy) phenylazanediyl)diacetic acid |
| APTRA | 2-carboxymethoxy-aniline-N,N-diacetic acid |
| BAPTA | 1,2-bis(-2-aminophenoxy)ethane- N,N,N',N'-tetraacetic acid |
| HIDA | N-(2-hydroxyethyl)iminodiacetic acid |
| Carboxyglutamate | 3-Aminopropane-1,1,3-tricarboxylic acid |

(CGlu)
citrate
arylazo chromotopic acid derivatives,
beta-diketone (crown-ether) derivatives,
mono-, di-, or tri-pyridyl aniline derivatives,
mono-, di-, or tri-pyridyl amine derivatives,
trimethylphenylammonium derivatives
and their hydrates, salts, solutions, stereoisomers.

A preferred compound with at least one metal-sensitive chemical shift is preferably selected from: pyro-EGTA, EGTA, EDTA or BAPTA, or a derivative or analog thereof.

According to the invention, a "derivative" or "analog" of pyro-EGTA, EGTA, EDTA, AATA, APTRA, BAPTA, HIDA, citrate, carboxyglutamate (CGlu), arylazo chromotopic acid, beta-diketone (crown-ether), mono-, di-, or tri-pyridyl aniline, mono-, di-, or tri-pyridyl amine, trimethylphenylammonium is preferably selected from
- acids, amides, esters, ethers, pyridine, ketone,
- fluorinated analogs,
including perdeuterated analogs, ¹⁸F radioisotopes,
and their hydrates, salts, solutions, stereoisomers.

Perdeuterations or perdeuterated analogs are preferably to increase T₁ times.

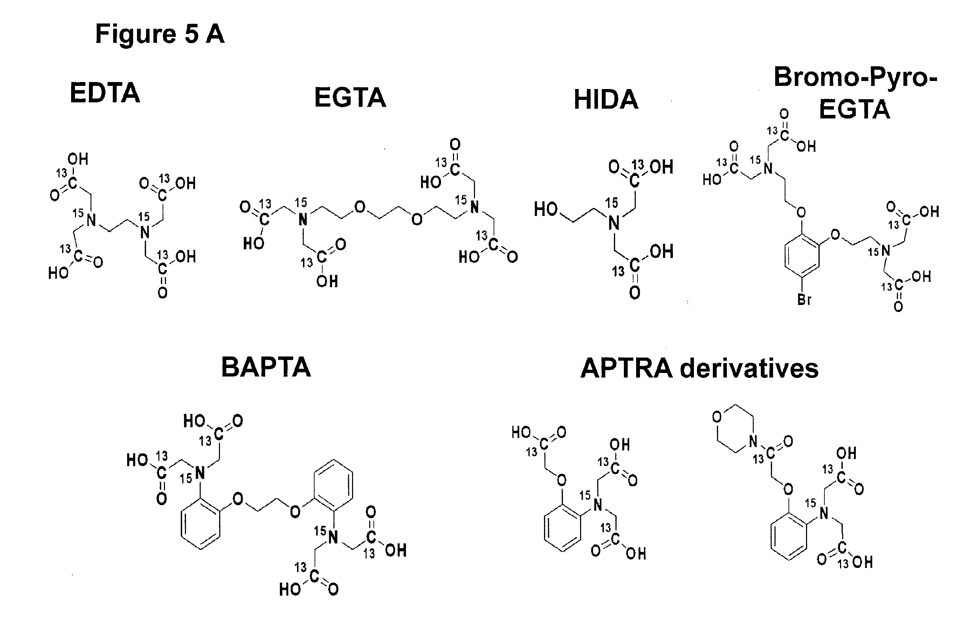

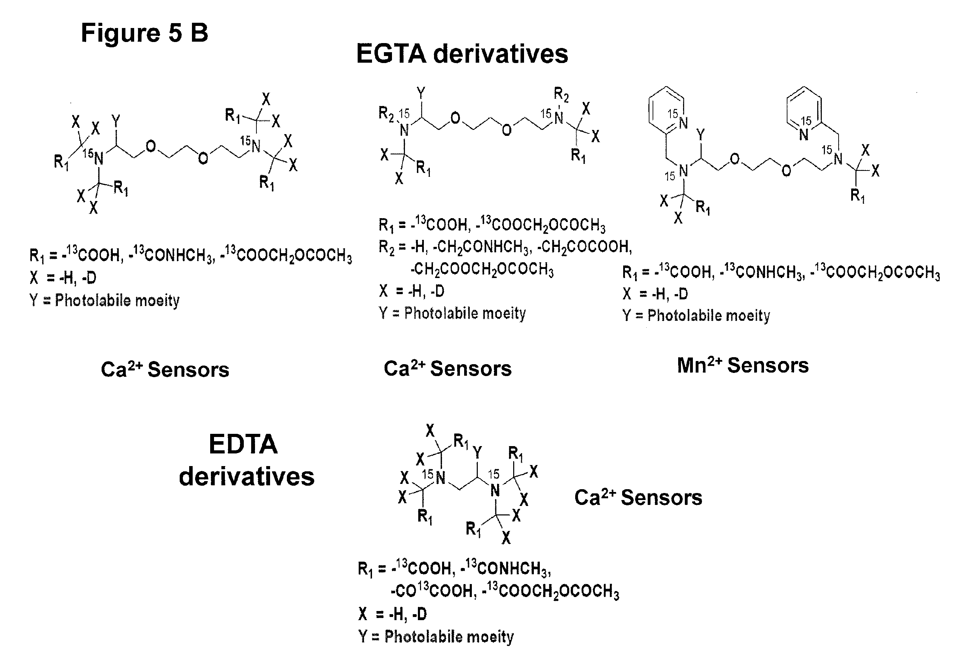

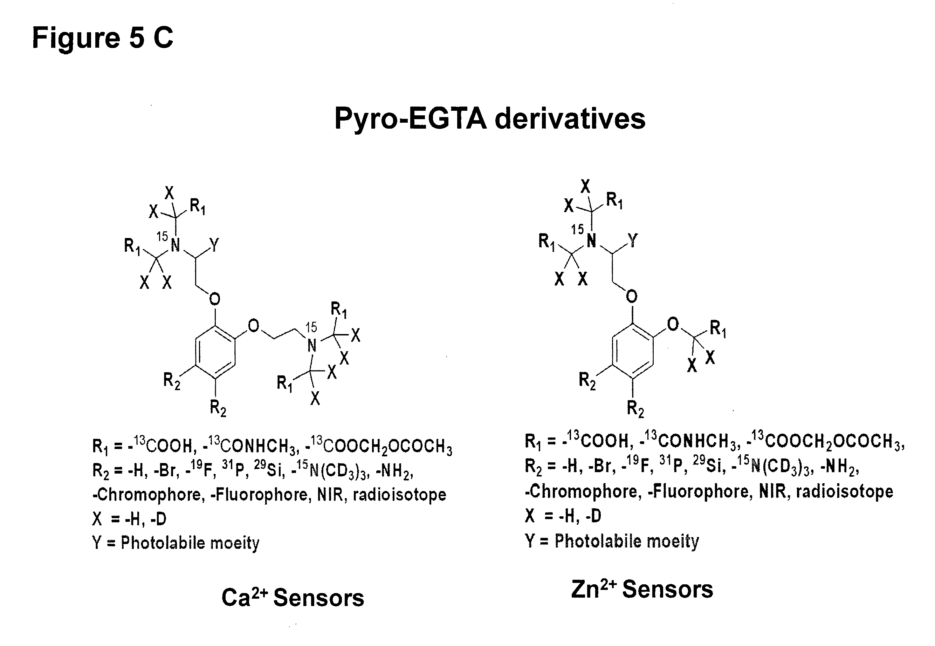

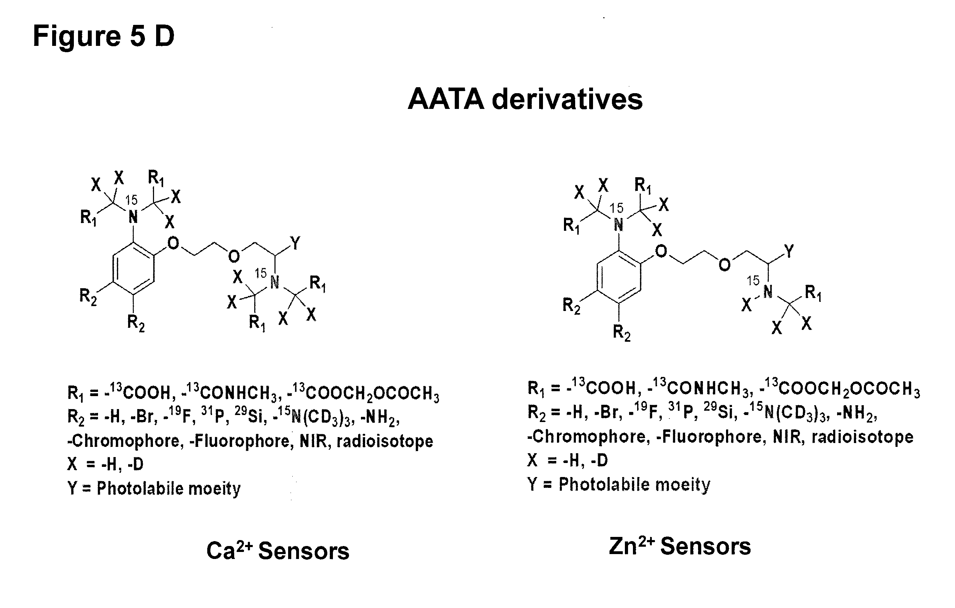

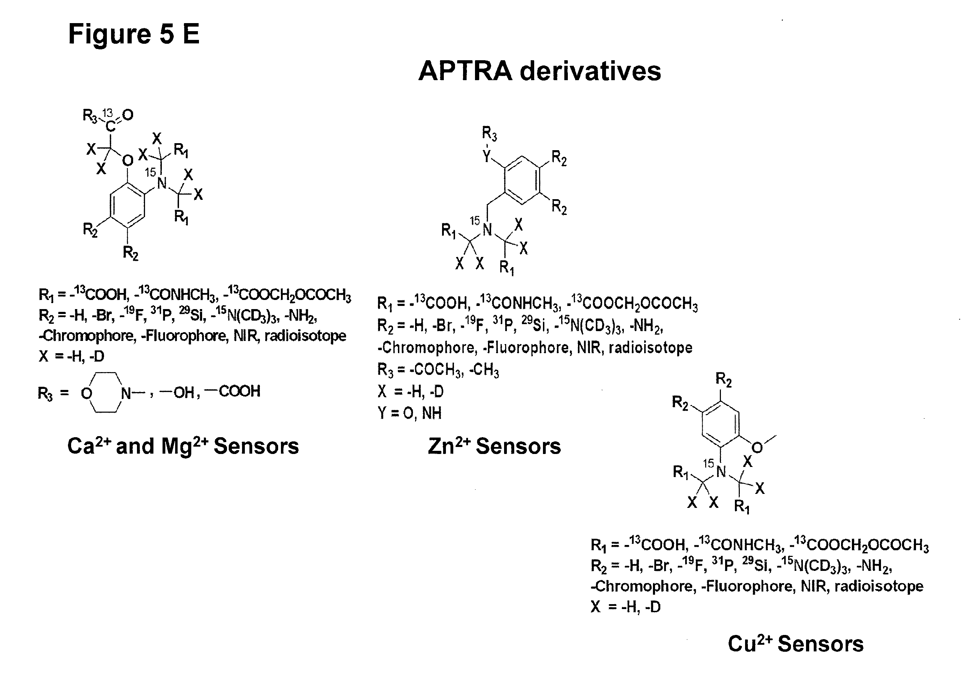

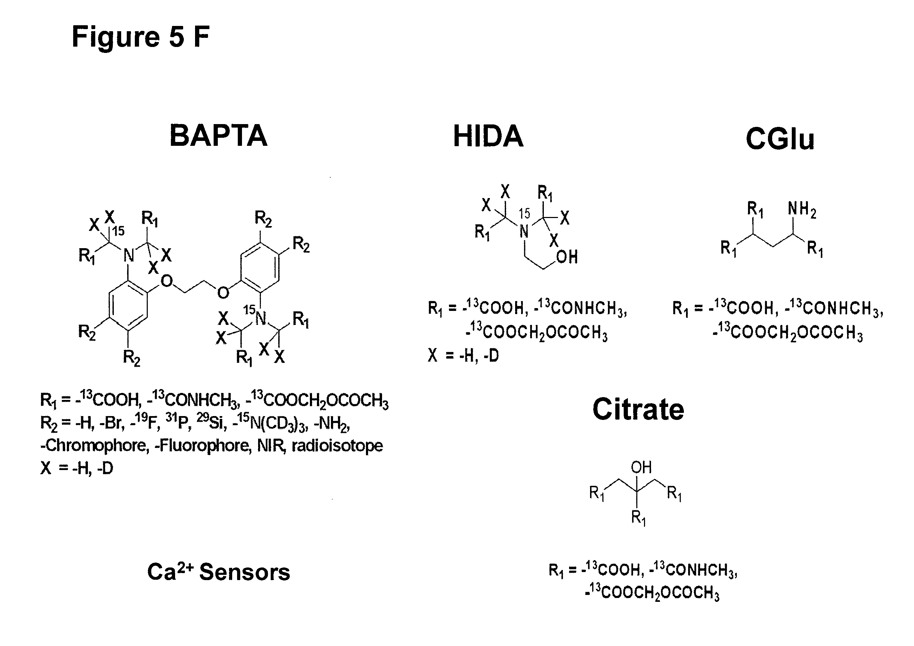

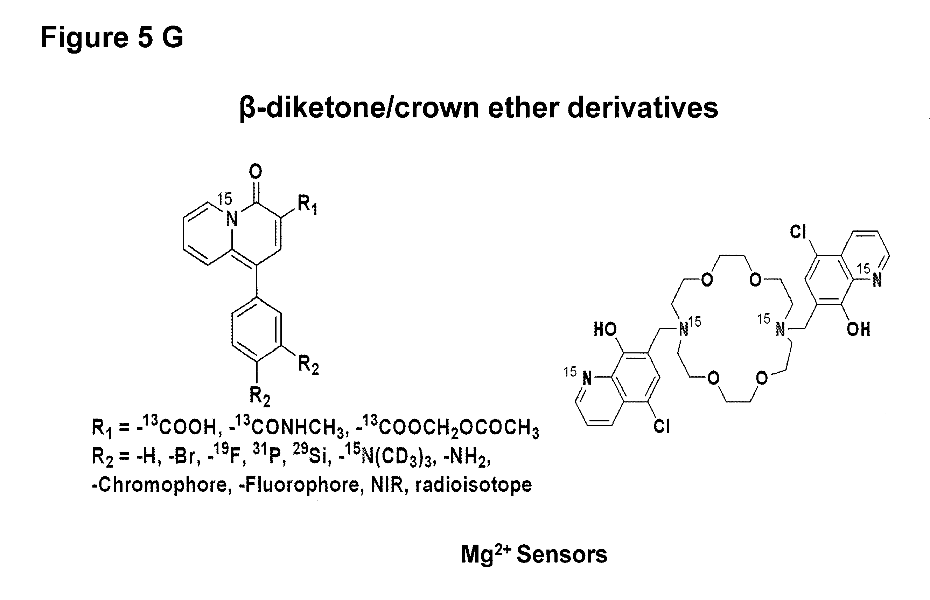

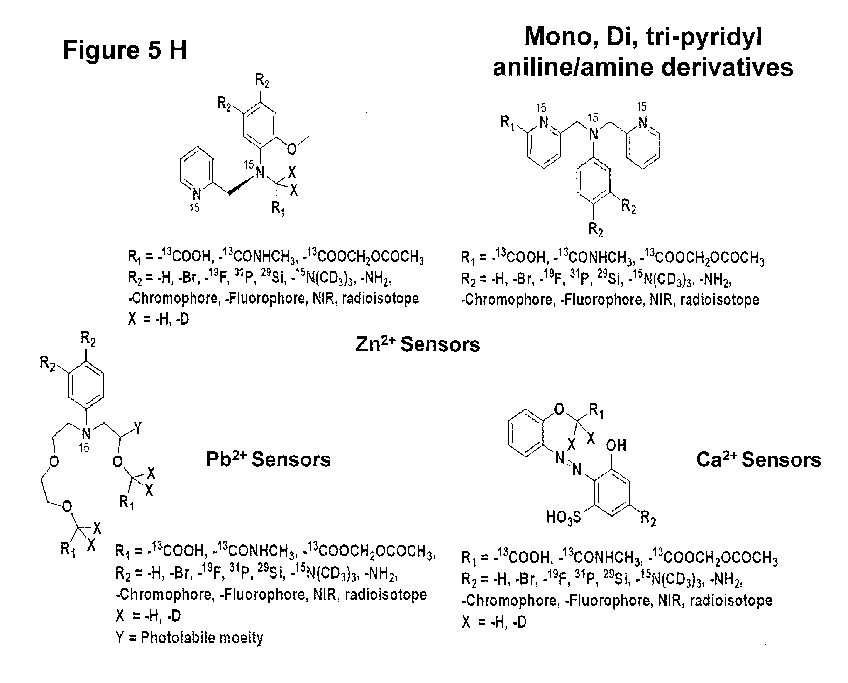

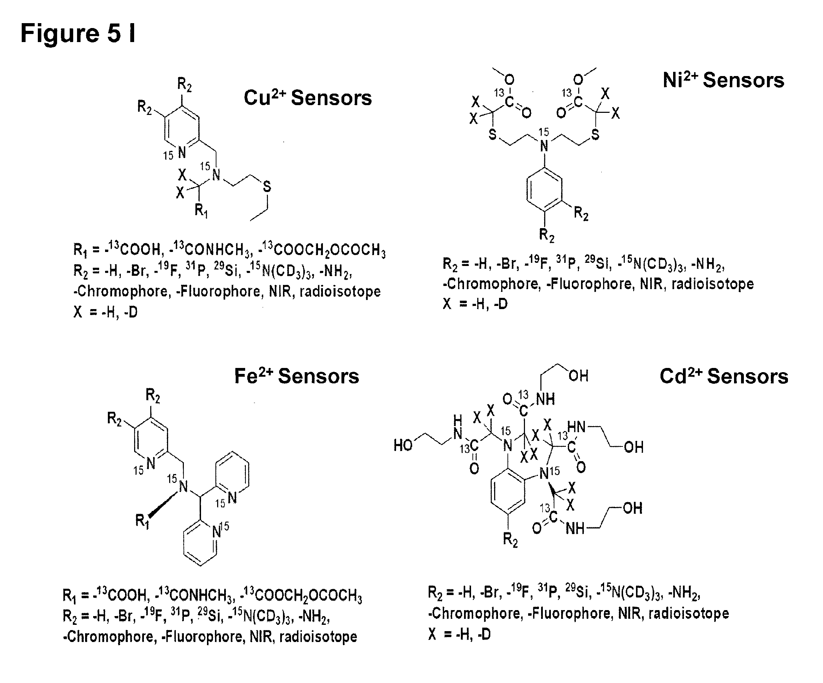

For examples, see Figure 5, wherein such possible perdeuterations to increase T₁ times are indicated by the symbol X.

Further preferred derivatives are compounds comprising at least one carboxylate, amide, nitrogen, oxygen group involved in coordinating of metals, such as shown as R₁ in Figures 5A-I, that may in addition have variable residues as indicated as R₂, R₃ and Y in Figures 5A-I.

For metal coordination, at least one carboxylate, amide, nitrogen or oxygen is required. A chemical shift can preferably be obtained either via a ¹³C, ¹⁵N or ¹⁷O as part of the metal-coordination site or via electron transfer to ¹H, ¹³C ,¹⁵N, ²⁹Si, ³¹P, ¹⁹F, ⁸⁹Yi residing e.g. on the meta or para position in pyro-EGTA, AATA, APTRA or BAPTA.

In one embodiment, the compound according to the present invention comprises *further component(s).*

Preferably, the further component(s) is/are selected from:
- label(s)
   such as fluorophore(s), chromophore(s), NIR dye(s),
   (*e.g.* cyanines for example indocyanine green (ICG) and derivatives, Bodipy and derivatives, squarine and derivatives, IR780 and derivatives and fluorescent proteins and chromoproteins)
   radioisotope(s);
- photo- and/or thermolabile moiety/moieties
   such as 6-nitroveratroyloxycarbonyl group (NVOC)
   (see *e.g.* Ellies-Davies, 2008);
- macrocycle
   preferably to stably hold a metal
   such as to generate a stable chelate of ⁸⁹Y and lanthanides;
- nanostructure(s),
   including magnetic nanoparticles and ultrasound contrast agents,
   and/or
- radiocontrast agent(s).

The further component(s) is/are preferably covalently attached via functional or anchoring group(s).

For example, the compounds / metal-specific sensors of the present invention can be modified such that bioconjugation/attachment of further component(s) can be easily achieved via suitable functional or anchoring group(s).

Figures 5 A-I shows examples of possible modifications.
Such as:
(i) via amine chemistry, preferably with photolabile moieties/groups such as 6-nitroveratroyloxycarbonyl group (NVOC)
   The photolabile moieties/groups can be connected to an α-carbon of the amine groups to get photolabile metal sensors. After photocleavage, the metal will be released non-reversibly. This is *e.g.* of interest to prevent contribution of non-signal generating compounds (*e.g.* after a time interval much longer than the relaxation time has passed) to the metal chelation capacity, *e.g.* if repeated measurements with hyperpolarized compounds are to be performed.
(ii) Furthermore, fluorophores , chromophores, radioisotopes can be connected to the chelators/ compounds / metal-specific sensors of the present invention
   e.g. via C-C, C-N or C-O,
   to *e.g.* validate their localization (measure their concentration for ratiometric approaches) by fluorescent microscopy. If the fluorophore is attached close to an aromatic ring, e.g. via C-C, C-N or C-O, chemical-shift inducing metal binding can also lead to simultaneous changes in fluorescence allowing for bimodal detection via MRI and fluorescence.

### - Metal-sensitivity

Preferably, the compound according to the present invention is labeled with ¹H, ¹³C, ¹⁵N, ¹⁹F, ²⁹Si, ³¹P, ⁸⁹Y or combinations thereof.

More preferably, the compound exhibits at least one metal-sensitive chemical shift of the ¹H, ¹³C, ¹⁵N, ¹⁹F, ²⁹Si, ³¹P and/or ⁸⁹Y nuclei.

More preferably, the compound according to the present invention exhibits a chemical shift that is specific for the particular metal, such that in one embodiment more than one metal (such as two, three, four or more) can be detected in parallel / at the same time.

Preferably, the compound exhibits at least one metal-sensitive chemical shift sensitive in the physiological and/or pathological metal concentration range.

A "physiological pH" or "physiological pH range" refers to pH ranges from about 5 to about 9, preferably a "physiological pH" or "physiological pH range" is from about pH 6 to about 8.

In a preferred embodiment, the compound is hyperpolarized.

Hyperpolarization of NMR active ¹³C, ¹⁵N nuclei may be achieved by different methods, which are for instance described in WO 98/30918, WO 99/24080 and WO 99/35508, and hyperpolarization methods are polarization transfer from a noble gas, "brute force", spin refrigeration, the parahydrogen method (ParaHydrogen Induced Polarisation (PHIP)) and Dynamic Nuclear Polarization (DNP).

Preferably, the hyperpolarization is done by Dynamic Nuclear Polarization (DNP).

The term "hyperpolarized" refers to a nuclear polarization level in excess of 0.1%, more preferred in excess of 1% and most preferred in excess of 10%. The level of polarization may for instance be determined by solid state NMR measurements, such as in solid hyperpolarized pyro-EGTA, EGTA, EDTA, AATA, APTRA, BAPTA, HIDA, citrate, carboxyglutamate (CGlu), arylazo chromotopic acid, beta-diketone (crown-ether), mono-, di-, or tri-pyridyl aniline, mono-, di-, or tri-pyridyl amine, trimethylphenylammonium (or other compounds, such as derivatives or analogs thereof), e.g. obtained by dynamic nuclear polarization (DNP) of pyro-EGTA, EGTA, EDTA, AATA, APTRA, BAPTA, HIDA, citrate, carboxyglutamate (CGlu), arylazo chromotopic acid, beta-diketone (crown-ether), mono-, di-, or tri-pyridyl aniline, mono-, di-, or tri-pyridyl amine, trimethylphenylammonium (or other compounds, such as derivatives or analogs thereof).
The solid state NMR measurement preferably consists of a simple pulse-acquire NMR sequence using a low flip angle. The signal intensity of the hyperpolarized nucleus in the NMR spectrum is compared with signal intensity in a NMR spectrum acquired before the polarization process. The level of polarization is then calculated from the ratio of the signal intensities before and after polarization. In a similar way, the level of polarization for dissolved hyperpolarized pyro-EGTA, EGTA, EDTA, AATA, APTRA, BAPTA, HIDA, citrate, carboxyglutamate (CGlu), arylazo chromotopic acid, beta-diketone (crown-ether), mono-, di-, or tri-pyridyl aniline, mono-, di-, or tri-pyridyl amine, trimethylphenylammonium (or other compounds, such as derivatives or analogs thereof) may be determined by liquid state NMR measurements. Again the signal intensity of the dissolved hyperpolarized pyro-EGTA, EGTA, EDTA, AATA, APTRA, BAPTA, HIDA, citrate, carboxyglutamate (CGlu), arylazo chromotopic acid, beta-diketone (crown-ether), mono-, di-, or tri-pyridyl aniline, mono-, di-, or tri-pyridyl amine, trimethylphenylammonium (or other compounds, such as derivatives or analogs thereof) is compared with the signal intensity before polarization. The level of polarization is then calculated from the ratio of the signal intensities before and after polarization.

In one embodiment, the ¹H, ¹³C, ¹⁵N, ¹⁹F, ²⁹Si, ³¹P and/or ⁸⁹Y nuclei belonging to the metal-sensitive and metal-specific chemical shift(s) of the compound of the present invention exhibit(s) a long longitudinal relaxation time T₁ and/or an optimized dynamic range of the metal concentration of interest.

### - Chemical shift reference

Preferably, a reference chemical shift which is metal-insensitive, *i.e.* not metal-sensitive and, thus, exhibits no change in chemical shift upon change of metal concentration, is required. This is typically provided in the form of a further compound, the "reference compound", that is added to or also present in a sample.

Alternatively, a chemical shift with a different correlation between the metal concentration and the chemical can serve as a reference. This may, *e.g.,* be a chemical shift within the compound according to the present invention.

The reference chemical shift can be an endogenous reference or an exogenous reference or a chemical shift of the compound itself or its metabolites.

In one embodiment, the compound with one or more metal-sensitive chemical shifts of the invention furthermore exhibits at least one chemical shift that is not metal-sensitive, preferably at least one metal-insensitive ¹H and/or ¹³C and/or ¹⁵N and/or ¹⁹F and/or ²⁹Si and/or ³¹P and/or ⁸⁹Y chemical shift. (endogenous reference).

In one embodiment, a reference compound is used. The reference compound is a compound which does not exhibit metal-sensitive shift(s) (exogenous reference).

Preferably the reference compound is labeled with ¹H and/or ¹³C and/or ¹⁵N and/or ¹⁹F and/or ²⁹Si and/or ³¹P and/or ⁸⁹Y-labelled and preferably exhibits at least one metal-insensitive chemical shift.

A preferred reference compound (for ¹³C) is ¹³C methanol or ¹³C urea.

As discussed above, the present invention provides an imaging medium, comprising at least one compound with at least one metal-sensitive chemical shift as defined herein, optionally, pharmaceutically acceptable carriers and/or excipients, such as an aqueous carrier, like a buffer.

Preferably, the imaging medium is a magnetic resonance (MR) imaging medium.

The term "imaging medium" refers to a liquid composition comprising at least one compound with one or more metal-sensitive chemical shifts of the present invention (such as hyperpolarized compounds as listed in Figure 5, including pyro-EGTA, EGTA, EDTA, AATA, APTRA, BAPTA, HIDA, citrate, carboxyglutamate (CGlu), arylazo chromotopic acid, beta-diketone (crown-ether), mono-, di-, or tri-pyridyl aniline, mono-, di-, or tri-pyridyl amine, trimethylphenylammonium (or other compounds, such as derivatives or analogs thereof) as the MR active agent. The imaging medium according to the invention may be used as imaging medium in MR imaging or as MR spectroscopy agent in MR spectroscopy. The imaging medium according to the invention may be used as imaging medium for *in vivo* MR imaging and/or spectroscopy, *i.e.* MR imaging and/or spectroscopy carried out on living human or non-human animal beings. Further, the imaging medium according to the invention may be used as imaging medium for in vitro MR imaging and/or spectroscopy, *e.g.* for determining metal concentration and/or metal concentration changes in cell cultures or *ex vivo* tissues. Cell cultures may be derived from cells obtained from samples derived from the human or non-human animal body, like for instance blood, urine, stool, semen, saliva or mucous fluids, while *ex vivo* tissue may be obtained from biopsies or surgical procedures.

In one embodiment, the imaging medium preferably comprises in addition to the MR active agent an aqueous carrier, preferably a physiologically tolerable and pharmaceutically accepted aqueous carrier, like water, a buffer solution or saline. Such an imaging medium may further comprise conventional pharmaceutical or veterinary carriers or excipients, *e.g.* formulation aids such as are conventional for diagnostic compositions in human or veterinary medicine.

In one embodiment, the imaging medium preferably comprises in addition to the MR active agent a solvent which is compatible with and used for in vitro cell or tissue assays, for instance DMSO or methanol or solvent mixtures comprising an aqueous carrier and a nonaqueous solvent, for instance mixtures of DMSO and water or a buffer solution or methanol and water or a buffer solution.

Preferably, at least one compound with at least one or more metal-sensitive chemical shifts is used in concentrations of up to 1 M, preferably 0.1 to 100 mM, such as 10 to 80 mM, in the imaging medium.

As discussed above, the present invention provides a *biosensor* for determining metal concentrations and/or measuring metal concentration changes.

Said biosensor comprises:
(i) at least one compound with at least one metal-sensitive chemical shift according to the present invention,
(ii) optionally, a reference compound,
(iii) optionally, one or several pharmaceutically acceptable carriers and/or excipients.
   Said biosensor can optionally further comprise
(iv) label(s) (such as fluorophore(s), chromophore(s), NIR dye(s), radioisotope(s))
   photo- and/or thermolabile moiety/moieties
   macrocycle
   nanostructure(s),
   and/or
   radiocontrast agent(s)
   as described above.

In one embodiment, the reference compound is a compound which does not exhibit metal-sensitive chemical shift(s) in an NMR spectrum (exogenous reference chemical shift, as described above).

Preferably, the reference compound is ¹H and/or ¹³C and/or ¹⁵N and/or ¹⁹F and/or ²⁹Si and/or ³¹P and/or ⁸⁹Y-labelled,
and preferably exhibits at least one metal-insensitive ¹H and/or ¹³C and/or ¹⁵N and/or ¹⁹F and/or ²⁹Si and/or ³¹P and/or ⁸⁹Y chemical shift.

Alternatively, a chemical shift with a different correlation between metal concentration and chemical shift can serve as a reference.

A preferred reference compound is ¹³C-urea or ¹³C-methanol.

For example, the reference compound is obtained in that a substance or compound (such as urea) is co-polarized at the same time when the compound with one or more metal-sensitive chemical shifts of the invention is hyperpolarized.

Preferably, the at least one compound with at least one/one or more metal-sensitive chemical shift is used in concentrations of up to 1 M, preferably 0.1 to 100 mM, such as 10 to 80 mM, in the biosensor.

### Imaging and medical uses

As discussed above, the present invention provides the compound with at least one metal-sensitive chemical shift of the present invention (the imaging medium of the present invention) or the biosensor of the present invention for use in *in vivo* magnetic resonance imaging (MRI), magnetic resonance spectroscopy (MRS) or magnetic resonance tomography (MRT).

As discussed above, the present invention provides the compound with at least one metal-sensitive chemical shift of the present invention or the biosensor of the present invention for use in absorbance/transmission, reflection, fluorescence or optoacoustic measurements and imaging.

In one embodiment, the magnetic resonance readout can be combined with photon-dependent imaging via transmission, fluorescence, or optoacoustic imaging techniques detecting a chromophore inserted.

As used herein "optoacoustic or photoacoustic" measurements or imaging refer to the detection of the mechanical waves generated by the optoacoustic, or photoacoustic effect

### (Ntziachristos et al., 2010)

As used herein "fluorescent or absorption" measurements or imaging refer to the detection of fluorescent and/or absorbed photons for point measurements or spatiotemporally resolved measurements/imaging.

As discussed above, the present invention provides the compound with at least one metal-sensitive chemical shift of the present invention or the biosensor of the present invention for use in diagnosing and/or monitoring treatment of diseases causing changes in metal concentrations.

Thereby, the progress of a disease and/or the treatment of a disease can be monitored.

Preferably, a "disease causing changes in metal concentrations" is selected from:
(1) diseases in which calcium signaling is affected
   as in:
   neuropsychiatric diseases, such as epilepsy,
   stroke,
   brain damage,
   neurodegenerative diseases,
   states of altered neuronal processing, such as sleep, coma, anesthesia, intoxication,
   cardiovascular diseases, such as arrhythmias, cardiac ischemia and myocardial infarct,
   neuromuscular or muscular diseases,
(2) diseases in which calcium and/or magnesium uptake, storage, utilization or excretion is affected
   as in
   endocrinological conditions, such as hyper/hypoparathyroidism, malnutrition and gastrointestinal diseases, such as malabsorption and
   diarrhea e.g. due to alcoholism,
   bone-related diseases, such as osteoporosis,
   kidney diseases and treatment with diuretics,
   osteoclastic processes of tumors or infections,
   diseases in which calcification in tissues occurs, such as cancers,
   including breast cancer,
   atherosclerotic alterations or inflammatory processes,
   due to other medical treatments,
(3) diseases in which iron uptake, storage, utilization or excretion is affected
   as in
   iron-deficit caused by *e.g.* malnutrition or blood loss, or anemia including genetically-caused anemias, infections and inflammation, neurodegenerative diseases, medical treatments
(4) diseases in which uptake, storage, utilization or excretion of other metals is affected
   as in
   neurodegenerative diseases (*e.g.* zinc, copper), or
   metal storage diseases, such as Wilson's disease,
   tumors, such as melanomas,
   medical treatments,
(5) diseases caused by metal intoxications
   with *e.g.* manganese, nickel, cobalt, lead, mercury.

Preferably, the imaging is real-time.

Preferably, the uses comprise the resolution of the spatiotemporal metal distribution, (such as spatiotemporal resolution to repeatedly capture spatiotemporal metal distributions),

preferably, comprising the use of frequency encoding techniques, such as all methods of chemical shift imaging (CSI) and phase sensitive encodings of chemical shifts (as *e.g.* used in non-invasive spatially resolved temperature measurements using changes in proton resonance frequencies (0.01 ppm/°C)). See Rieke *et al.,* 2004.

Preferably, the use comprises monitoring extra- and intracellular calcium signaling and (treatments of) metal metabolism and intoxication in preclinical animal models, such as zebrafish, rodents and non-human primates.

In one embodiment, multimodal imaging via absorbance, fluorescence or optoacoustics, is used in addition, and/or the metal coordination and pharmacokinetics of the biosensor are controlled via breaking the photolabile and/or thermolabile moieties, if present and as specified herein above.

As discussed above, the present invention provides the compound with at least one metal-sensitive chemical shift of the present invention as metal sensor for *in vitro* or *ex-vivo* NMR spectroscopy.

Preferably, the use comprises response-to-treatment monitoring of treatments applied to cell lines, tissue cultures or explanted tissue.

Preferably, the use comprises monitoring extra- and intracellular calcium signaling and (treatments of) metal metabolism and toxicity in tissue culture and cell lines.

*Methods for determining metal concentration and*/*or measuring metal concentration changes* As discussed above, the present invention provides an *in vitro* as well as an *in vivo* method for determining metal concentration and/or measuring metal concentration changes.

### Said in vitro method comprises the steps of

(i) providing a sample,
(ii) adding a compound with at least one metal-sensitive chemical shift of the present invention or a biosensor of the present invention to the sample,
(iii) performing magnetic resonance imaging (MRI) or magnetic resonance spectroscopy (MRS) and thereby determining the spatial distribution of metal concentration or metal concentration changes of or in the sample
   (1) by obtaining a chemical shift difference between at least one metal-sensitive chemical shift of the compound and a metal-independent chemical shift, such metal-independent chemical shift acting as a reference chemical shift, or
   (2) by measurement of the absolute chemical shift, or
   (3) by measuring chemical shift differences involving at least one metal-sensitive shift, or
   (4) by obtaining additional measurements of metal-insensitive signals that relate to the concentration of the sensor, for instance based on radioactivity, absorbance, fluorescence or optoacoustic signal from the metal-sensor containing the appropriate radioactive moiety, fluorophore/chromophore,
preferably measuring simultaneously the spatiotemporal distribution of several metals at once based on the metal-specific chemical shifts, *e.g.* the Mg²⁺, Ca²⁺ and Zn²⁺ simultaneously with parallel determination of pH.

Preferably, the sample is a cell culture sample, such as derived from a human or non-human body, ex vivo tissue, cell culture.

Preferably, step (iii) is carried out in an MRI scanner machine with MRS or MRSI capabilities or in a NMR spectrometer (such as via a micro imaging head).

In one embodiment, the metal-independent chemical shift (acting as a reference chemical shift) is from the same compound, *i.e.* the compound with at least one metal-sensitive chemical shift (endogenous reference chemical shift, as described above), or from another substance (exogenous reference chemical shift, as described above), and is used as a metal-independent reference.

Alternatively, a chemical shift with a different correlation between metal concentration and chemical shift can serve as a reference.

Said *in vivo* method of the present invention comprises the steps of
(i) applying or administering a compound with at least one metal-sensitive chemical shift of the present invention or a biosensor of the present invention to the body of a patient or non-human animal,
(ii) performing magnetic resonance imaging (MRI) and thereby determining one or several metal concentrations or metal concentration changes of or in the body of said patient or non-human animal
   (1) by correcting a spatiotemporally resolved metal-sensitive signal for differences in local and time-dependent signal intensities, *e.g.* due to differences in the local concentration of the metal sensors, and
      by obtaining the chemical shift and/or its integrated peak area and/or peak amplitude from at least one metal-sensitive chemical shift and a metal-insensitive reference in the form chemical shift and/or integrated peak area from at least one metal-insensitive chemical shift or a function of metal-sensitive shifts,
      or
   (2) by measurement of the absolute chemical shift, integrated peak area or peak amplitude,
      or
   (3) by measuring a metal-insensitive MRI signal from another nucleus such as proton via a lanthanide, ¹H and/or ¹³C and/or ¹⁵N and/or ¹⁹F and/or ²⁹Si and/or ³¹P and/or ⁸⁹Y, or
   (4) by combination with a PET measurement *e.g.* via ¹⁸F or fluorescence or optoacoustics *e.g.* via a chromophore or fluorophore,

Preferably, the *in vivo* method is a real-time method.

In one embodiment, the patient is human.

Preferably, the patient can be diagnosed with a disease causing changes in metal concentrations or the treatment of a disease causing changes in metal concentrations can be monitored.

Preferably, a "disease causing changes in metal concentrations" is preferably selected from the diseases as defined above.

In one embodiment, the metal-independent chemical shift (acting as a reference chemical shift) is from the same compound, *i.e.* the compound with at least one metal-sensitive chemical shift (endogenous reference chemical shift, as described above), or from another substance (exogenous reference chemical shift, as described above), and is used as a metal-independent reference.

Alternatively, a chemical shift with a different correlation between metal concentration and chemical shift can serve as a reference.

Preferably, the *in vitro* and/or the *in vivo* method comprises the resolution of the spatial metal distribution and, thus, obtaining spatially resolved NMR spectra,
preferably, comprising the use of frequency encoding techniques, such as all methods of chemical shift imaging (CSI) and phase sensitive encodings of chemical shifts (as *e.g.* used in non-invasive spatially resolved temperature measurements using changes in proton resonance frequencies (0.01 ppm/0 C) (see Rieke *et al.,* 2004),
as well as indirect methods based on detecting protons in proximity of the other nucleus, such as ¹³C or ¹⁵N proton-observed carbon-edited (POCE) sequences and its analogs, as well as CEST or Magnetization Transfer (MT) methods.

### Methods for diagnosing and/or monitoring treatment

As discussed above, the present invention provides a method of diagnosing and/or monitoring treatment of a disease causing changes in metal concentrations.

### Said method comprises the steps of

(i) applying or administering a compound with at least one metal-sensitive chemical shift of the present invention or a biosensor of the present invention to the body of a patient or non-human animal,
(ii) performing magnetic resonance imaging (MRI) or magnetic resonance spectroscopy (MRS) and thereby determining one or several metal concentrations or metal concentration changes of or in the body of said patient or non-human animal
   (1) by obtaining over time the chemical shift and/or its integrated peak area and/or its peak amplitude from at least one metal-sensitive chemical shift and a metal-insensitive reference in the form of a chemical shift and/or its integrated peak area and/or peak amplitude from at least one metal-insensitive chemical shift or a function of metal-sensitive shifts,
      or
   (2) by measurement of the absolute chemical shift, integrated peak area or peak amplitude,
      or
   (3) by measuring a metal-insensitive MRI signal from another nucleus such as proton via a lanthanide, ¹H and/or ¹³C and/or ¹⁵N and/or ¹⁹F and/or ²⁹Si and/or ³¹P and/or ⁸⁹Y, or
   (4) by combination with a PET measurement *e.g.* via ¹⁸F or fluorescence or optoacoustics *e.g.* via a chromophore or fluorophore, or CT via a radiocontrast agent, (iii) calculating metal concentration maps based on spatially resolved metal concentration values or metal concentration changes determined in step (ii),

Preferably, a "disease causing changes in metal concentrations" is preferably selected from the diseases as defined herein above.

Thereby, the progress of a disease and/or the treatment of a disease can be monitored.

Preferably, step (iii) preferably comprises
comparing said relative chemical shifts to predetermined or simultaneously measured calibration curves of the compound with at least one metal-sensitive chemical shift in solutions with known metal concentration or mixtures of metals with known concentrations.

In one embodiment, said method furthermore comprises
hyperpolarizing the compound with at least one metal-sensitive chemical shift before application or administration to the body of the patient.

Thereby, the compound is hyperpolarized by any hyperpolarization methods, such as dissolution Dynamic Nuclear Polarization (DNP) or ParaHydrogen Induced Polarisation (PHIP).

Preferably, the method comprises magnetic resonance tomography (MRT).

Preferably, the imaging is real-time.

Preferably, the method comprises the resolution of the spatial metal distribution and, thus, obtaining spatially resolved NMR spectra,
preferably, comprising the use of frequency encoding techniques, such as all methods of chemical shift imaging (CSI) and phase sensitive encodings of chemical shifts (as *e.g.* used in non-invasive spatially resolved temperature measurements using changes in proton resonance frequencies (0.01 ppm/0 C) (see Rieke *et al.,* 2004),
as well as indirect methods based on detecting protons in proximity of the other nucleus, such as ¹³C or ¹⁵N proton-observed carbon-edited (POCE) sequences and its analogs, as well as CEST or Magnetization Transfer (MT) methods.

### Further uses

As discussed above, the present invention provides the use of a compound of the present invention or the biosensor of the present invention in quality control of food or in the examination of plants and organisms,
or for monitoring of environmental resources, including air, water, and soil with respect to pollution and harmful metal concentrations.

### Derivatives of pyro-EGTA, AATA, APTRA and BAPTA

As discussed above, the present invention provides derivatives or analogs of pyro-EGTA, AATA, APTRA or BAPTA.

The derivatives or analogs of pyro-EGTA, AATA, APTRA or BAPTA of the present invention comprise a "modifying moiety" or "modifying moieties". Said modifying moiety/moieties or group(s) alter(s) the affinity for metals (and are thus referred to as "metal affinity modifying moiety" or "metal affinity modifying moieties").

Said modifying moiety/moieties is/are preferably selected from:
- halogen (*e.g.* Cl, Br, I),
- a hyperpolarizable nucleus (e.g. ¹⁹F, or ³¹P, ²⁹Si, ¹⁵N), or
- NH₂,

Suitable examples for said modifying moieties are shown in Figures 5B to 5I, see R₁ and R₂.

The derivatives are preferably compounds comprising at least one carboxylate, amide, nitrogen, oxygen group involved in coordinating of metals, such as shown as R₁ in Figures 5A-I, that may in addition have variable residues as indicated as R₂, R₃ and Y in Figures 5A-I.

For metal coordination, at least one carboxylate, amide, nitrogen or oxygen is required. A chemical shift can preferably be obtained either via a ¹³C, ¹⁵N or ¹⁷O as part of the metal-coordination site or via electron transfer to ¹H, ¹³C ¹⁵N, ²⁹Si, ³¹P, ¹⁹F, ⁸⁹Yi residing *e.g.* on the meta or para position in pyro-EGTA, AATA, APTRA or BAPTA.

For example,
- As shown in Figure 5C, for pyro-EGTA, suitable metal affinity modifying moieties or groups are *e.g.*
   ¹³COOH, ¹³CONHCH₃, ¹³COOCH₂OCOCH₃,
   Br, ¹⁹F, or ³¹P, ²⁹Si, ¹⁵N, ¹⁵N (CD₃)₃, NH₂
- As shown in Figure 5D, for AATA, suitable modifying moieties or groups are *e.g.*
   ¹³COOH, ¹³CONHCH₃, ¹³COOCH₂OCOCH₃,
   Br, ¹⁹F, or ³¹P, ²⁹Si, ¹⁵N, ¹⁵N (CD₃)₃, NH₂
- As shown in Figure 5E, for APTRA, suitable modifying moieties or groups are *e.g.*
   ¹³COOH, ¹³CONHCH₃, ¹³COOCH₂OCOCH₃,
   Br, ¹⁹F, or ³¹P, ²⁹Si, ¹⁵N, ¹⁵N (CD₃)₃, NH₂
- As shown in Figure 5F, for BAPTA, suitable modifying moieties or groups are *e.g.*
   ¹³COOH, ¹³CONHCH₃, ¹³COOCH₂OCOCH₃,
   Br, ¹⁹F, or ³¹P, ²⁹Si, ¹⁵N, ¹⁵N (CD₃)₃, NH₂

Preferred embodiments of the derivatives or analogs are Bromo-pyro-EGTA nad ¹⁹F-pyro-EGTA.

As discussed above, the present invention provides the derivatives or analogs of pyro-EGTA, AATA, APTRA or BAPTA of the present invention for use as metal biosensors, i.e. the uses and in the methods according to the present invention, as discussed herein above.

### Further description of preferred embodiments

The features of the metal biosensors of the present invention are:
a) chemical shift
   - metal-specific chemical shift
      - that is robust against pH changes
      - in the case of EDTA (and its derivatives), pH can be measured simultaneously with calcium
   - tunable sensitivity and specificity for different metals
   - compounds are hyperpolarizable to achieve stronger signals for *in vivo* applications
b) coupling of further component(s) is possible, such as via functional group(s):
   wherein such further components can be:
      - biomolecule(s),
      - photolabile moiety/moieties,
      - label(s): chromophore(s), fluorophore(s), NIR dye(s), radioisotope(s)

The inventors show that the metal-specific chemical shift of the metal biosensor compounds of the present invention, in particular or EDTA and EGTA, is independent of the pH shifts. In the case of EDTA, pH can be read out simultaneously with the metal content.

The inventors furthermore show that calcium can be detected quantitatively in the presence of another metal, namely magnesium, by the metal biosensor compounds of the present invention (in particular EDTA and EGTA) based on the metal-specificity of the chemical shift, in particular of EDTA and EGTA, Moreover, the apparent affinity of calcium in the presence of magnesium is reduced as is desired for detection of extracellular magnesium levels.

As discussed above, Nonaka *et al.* (2013) have recently published a ¹⁵N-containing compound, namely [¹⁵N, D₉]trimethylphenylammonium ([¹⁵N, D₉]TMPA) that can be hyperpolarized by DNP and exhibits a chemical shift of the ¹⁵N moiety when coupled to a metal chelator APTRA.

In distinction to Nonaka *et al.* (2013), the hyperpolarizable compounds disclosed in the present document exhibit
(1) a metal-specific, reversible chemical shift that e.g. allows for detection of calcium in the presence of equimolar concentrations of magnesium;
(2) robustness of metal-sensitive shift against pH changes with the ability to readout pH in parallel (*e.g.* as shown for ¹³C-EDTA),
(3) a wide range of tuned dissociation constants.

The following examples and drawings illustrate the present invention without, however, limiting the same thereto.

### BRIEF DESCRIPTION OF THE DRAWINGS

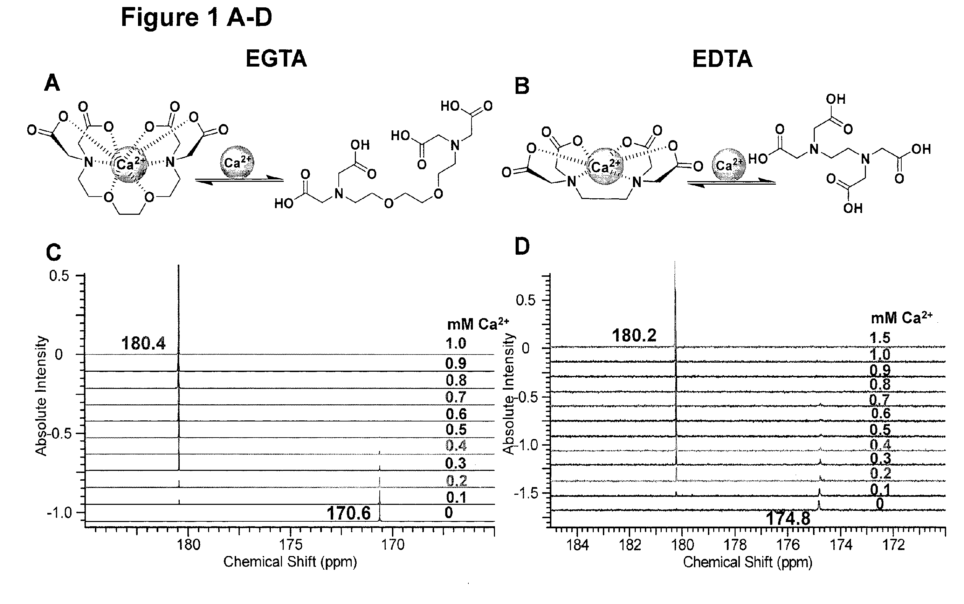

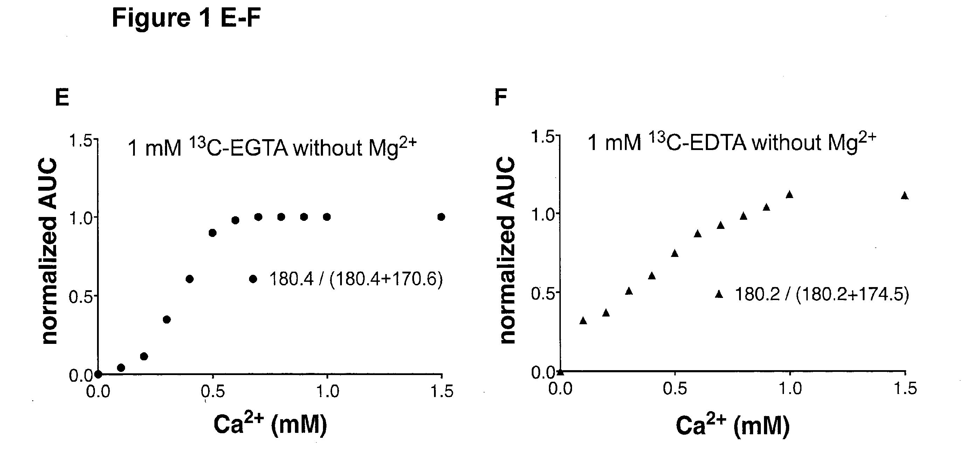

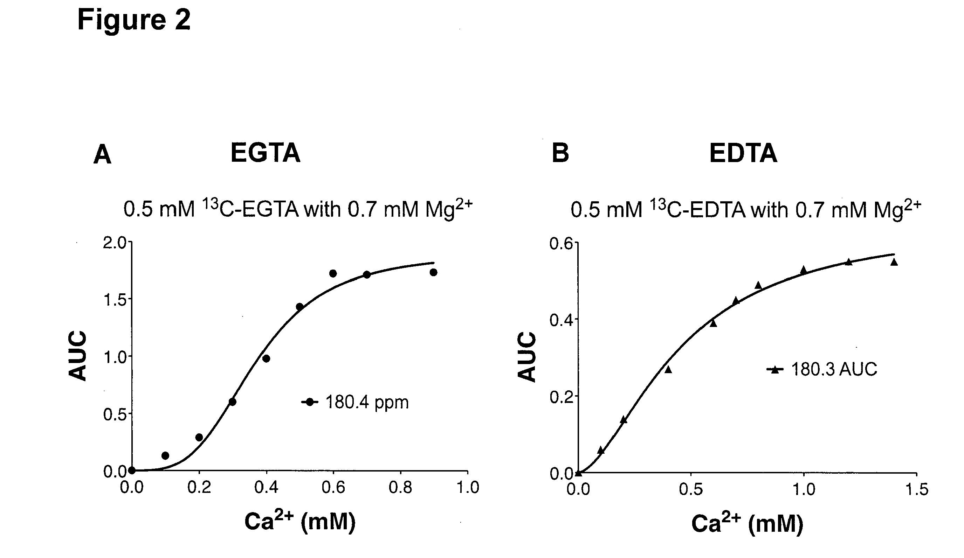

**Figure 1****.** *Calcium detection with calcium-specific biosensors.*
   **(A and B)** Scheme depicting the structure and calcium-coordination of ¹³C-EGTA and ¹³C-EDTA
   **(C and D)** ¹³C NMR spectra for 1 mM ¹³C-EGTA and ¹³C-EDTA with addition of 0-1.5 mM calcium chloride in 15 mM MOPS with H₂0/D₂0 (1:1). Increasing concentrations of calcium result in a peak at ~180 ppm with increasing amplitude (1 µL ¹³C-methanol was used as an internal reference; measurements were taken at 310 K).
   **(E and F)** Titration curves obtained from the spectra in C and D. The Area under the Curve (AUC) was taken for the chemical shift corresponding to the bound calcium (~180 ppm) and normalized against the sum of the shifts corresponding to calcium-bound and calcium-free chelator (170.6 ppm and 174.5 ppm respectively). The dissociation constant *(K_{D})* for ¹³C-EGTA was 0.3489 mM (0.3314 mM to 0.3664 mM, 95% confidence intervals) 0,5278 mM (0,05863 mM to 0,9970 mM, 95% confidence intervals) for ¹³C-EDTA
**Figure 2****.** *Calcium detection with calcium-specific biosensors in the presence of magnesium.*
   **(A and B)** Calcium titration curves obtained analogously to Figure 1 with 0.5 mM ¹³C-EGTA and ¹³C-EDTA in calcium free artificial cerebrospinal fluid (aCSF) containing 0.7 mM Mg²⁺ (0.5 µL ¹³C-MeOH was used as an internal reference) (310 K, 500 MHz (¹³C 125.83 MHz), pH 7.4). kD values were 0.3703 (0.3135 to 0.4272) for ¹³C-EGTA and 0.4589 (0.3607 to 0.5571) for ¹³C-EDTA.

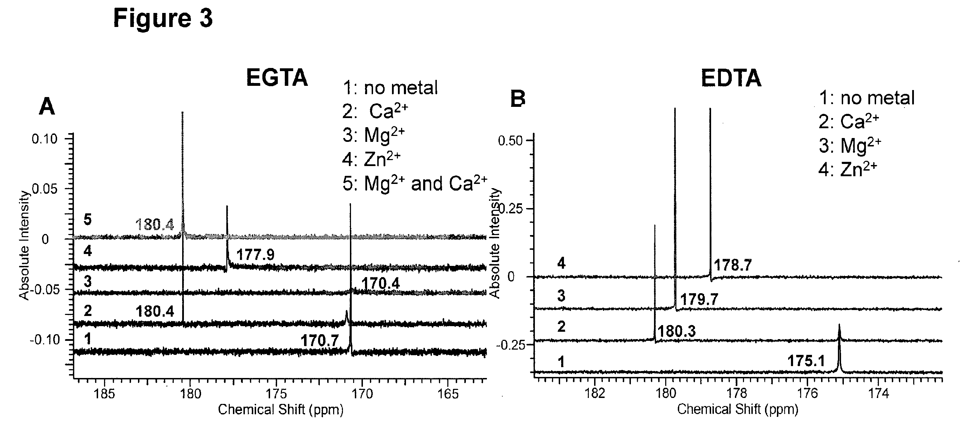

**Figure 3****.** *Metal-specific chemical shifts.*
   1 mM calcium, magnesium or zinc were added to 1 mM **(A)** ¹³C-EGTA and **(B)** C-EDTA and resulted in distinct chemical shift for each metal (15 mM MOPS in H20/D20 (1:1), 0.5 µL ¹³C-methanol was used as an internal reference; measurements were taken at 310 K, 500 MHz (¹³C 125.83 MHz), pH 7.4).

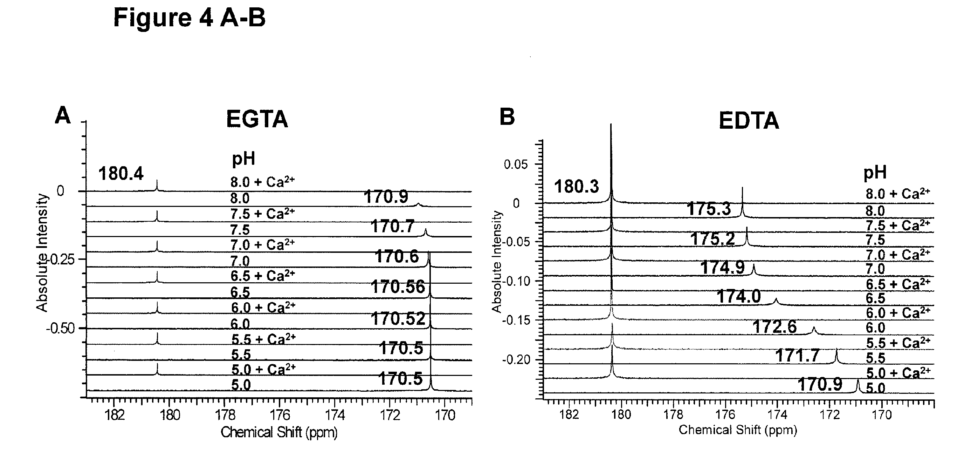

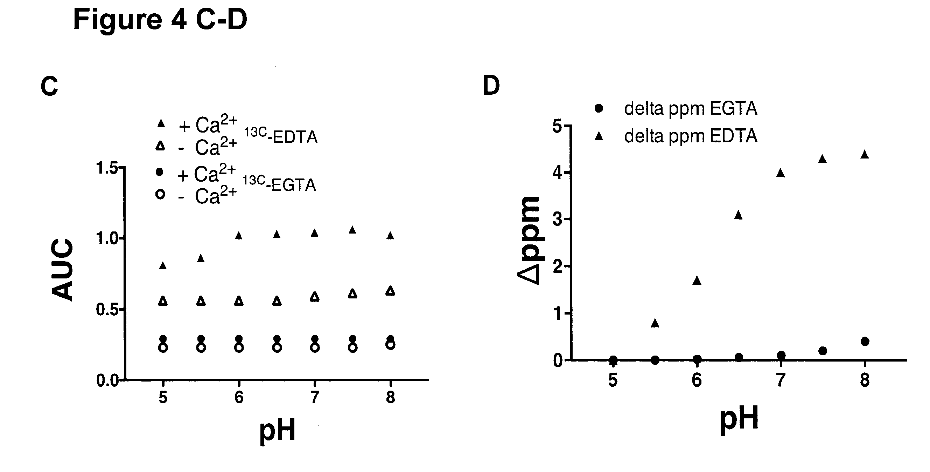

**Figure 4****.** *ph-dependence of the calcium sensors' signal.*
   **(A)** 1 mM ¹³C-EGTA and **(B)** ¹³C-EDTA in 15 mM MOPS buffer was adjusted to a pH range between 5 and 8. In half of the samples 1 mM calcium chloride was added. (0.5 µL ¹³C-methanol was used as an internal reference), measurements at 310 K, 500 MHz (¹³C 125.83 MHz), pH 7.4).
   **(C)** AUCs were computed for the peaks corresponding to calcium-free and calcium-bound compound respectively.
   **(D)** The difference in ppm as compared with pH 5 is plotted for both calcium sensors.
**Figure 5****.** *Molecular Structures of Metal Biosensors.*
   Scheme of the core structures (and their derivatives) of the metal sensors indicating the positions at which ¹³C and ¹⁵N, ²⁹Si, ¹⁹F, ⁸⁹Y atoms can be introduced.
   Modifications useful for bioconjugation or attaching chromophores, fluorophores or radioisotopes are enumerated as R_{1,2}. In Figures 5B-I, modifications of the metal-coordinating moieties that can alter affinities for metals are listed as options for R1,₂. Possible perdeuterations to increase T₁ times are indicated by the symbol X, photolabile moieties to influence the stability of the molecule as Y.
   **(A)** core structures **(B)** EGTA and EDTA derivatives, **(C)** pyro-EGTA derivatives, **(D)** AATA derivatives, **(E)** APTRA derivatives, **(F,G)** BAPTA, HIDA and CGlu and β-ketone/crown ether derivatives, **(H, I)** mono-, di-, tri-pyridyl aniline/amine derivatives.

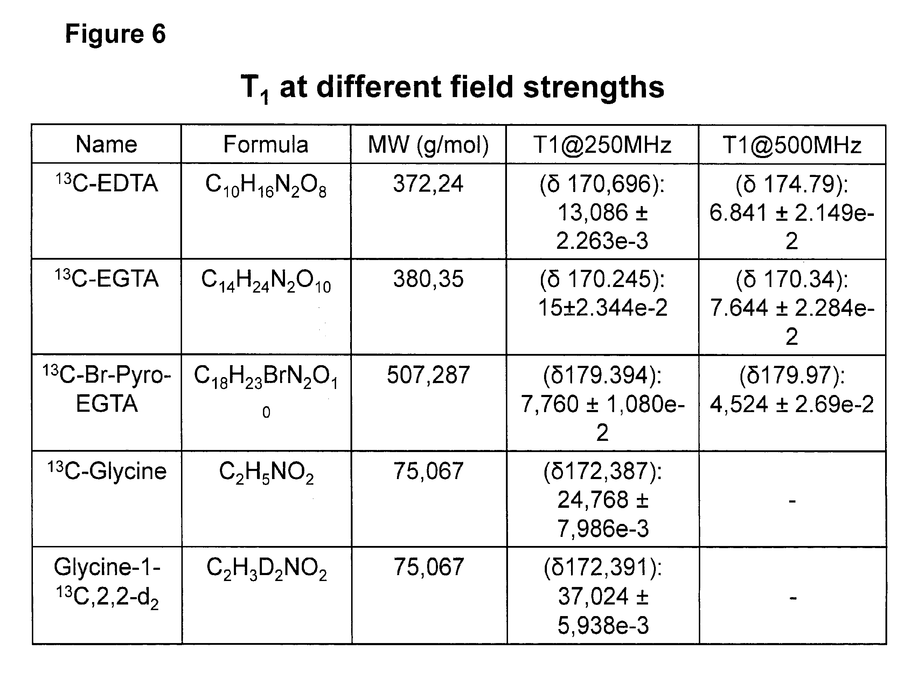

**Figure 6****.** *Selected T₁ values at different field strengths.*

**Table 1 Metal Biosensor Properties**

| **Name** | **Formula** | **MW (g/mol)** | **T₁ (sec) @ 500 MHz** | **Shift** δ **(ppm)** |
|---|---|---|---|---|
| **EDTA** | C₁₀R₁₆N₂O₈ | 372,24 | (δ 174.79): **6.841** ± 2.149e-2 +CaCl₂ (1:1)(δ 179.98): 5.595± 1.248e-2 | 5,2 |
| **Br-Pyro-EGTA** | C₁₈H₂₃BrN₂O₁₀ | 507,287 | (δ179.97): **4,524** (δ 147.84): 2.26 ± 2.26e-2 (δ 146.45): 2.395 ± 3.612e-2 | 0,8 |
| **EGTA** | C₁₄H₂₄N₂O₁₀ | 380,35 | (δ 170.34): **7.644** ± 2.284e-2 +CaCl₂ (1:1) (δ 180.13):5.481 ± 1.322e-2 | 9,8 |
| **APTRA** | C₁₂H₁₃NO₇ | 283,234 | (δ 179.60): **3.879** ± 4.33e-2 (δ 177.11): 5.561 ± 7.343e-2 (δ 150.74): 1.34 ± 5.55e-2 | not significant |
| **BAPTA** | C₂₂H₂₄N₂O₁₀ | 476,433 | (δ 178.86): **2.86** ± 1.58e-2 (δ 149.83): 2.16 ± 3.12e-2 (δ 140.06): 5.09 ± 5.81e-+CaCl₂ (δ 178.56): 1.979 ± 6.61e-2 | 0,3 |
| **HIDA** | C₆H₁₁NO₅ | 177,15 | (δ 170,306): **10,326** ± 5,807e-3 +CaCl₂ (1:1)(δ 170,725): 3,484± 4,819e-2 | 0,3 |
| **Carboxy - Glutamate (CGlu)** | C₇H₁₀O₆ | 191,14 | (δ 178,385): 9,677 ± 8,528e-2 (δ 177,944): 10,131± 9,985e-2 (δ 174,593): 10,751± 9,334e-2 | tbd |

### Binding affinities

| **Name** | **k_{D}-Ca** | **k_{D}-Mg** | **k_{D}-Zn** | **k_{D}-Cu** |
|---|---|---|---|---|
| **EDTA** | 1,09648E-11 | 2,04174E-09 | 3,16228E-17 | 1,58489E-19 |
| **Pyro-EGTA** | tbd. | tbd. | tbd. | tbd. |
| **EGTA** | 1E-11 | 6,30957E-06 | 1,25893E-13 | 1,99526E-18 |
| **APTRA** | 1,45E-03 | 1,80E-05 | - | - |
| **BAPTA** | 1,07E-07 | 1,70E-02 | 1,99526E-12 | 1,99526E-12 |

| **Name** | **k_{D}-Fe(II)** | **k_{D}-Fe(III)** | **k_{D}-Mn** | **k_{D}-Ni** |
|---|---|---|---|---|
| **EDTA** | 4,67735E-15 | 7,94328E-26 | 9,12011E-11 | 2,39883E-19 |
| **Pyro-EGTA** | tbd. | tbd. | tbd. | tbd. |
| **EGTA** | 1,20226E-12 | 3,16228E-21 | 7,94328E-13 | 1,58489E-12 |
| **APTRA** | - | - | - | - |
| **BAPTA** | - | - | 1,86209E-09 | - |

| **Name** | **k_{D}-Co(II)** | **k_{D}-Pb** | **k_{D}-Cd** | **k_{D}-Hg** |
|---|---|---|---|---|
| **EDTA** | 4,8978E-17 | 9,12011E-19 | 3,46737E-17 | 1,58489E-22 |
| **Pyro-EGTA** | tbd. | tbd. | tbd. | tbd. |
| **EGTA** | 5,01187E-13 | 9,12011E-19 | 1,99526E-17 | 7,58578E-24 |
| **APTRA** | - | - | - | - |
| **BAPTA** | 1,995E-9 | 5,01187E-12 | 6,30957E-13 | - |

### EXAMPLES

### EXAMPLE 1

### Materials and Methods

### 1.1 General

The chemicals and anhydrous solvents were purchased from commercial suppliers (Aldrich, Fluka, and VWR) and were used without further purification unless otherwise stated. Unless otherwise mentioned, all the reactions were carried out under an inert (nitrogen) atmosphere. All glassware was washed with a mixed acid solution and thoroughly rinsed with deionized, distilled water and pre-dried with a heat gun under vacuum. Ultra-pure deionized water (18 MΩ cm⁻¹) was used throughout.

### 1.2 Chromatography.

Flash column chromatography was performed by using flash silica gel 60 (70-230 mesh) from Merck. Thin layer chromatography (TLC) was performed on aluminum-backed silica gel plates with 0.2 mm thick silica gel 60 F₂₅₄ (E. Merck) using different mobile phases. The compounds were visualized by UV irradiation (254 nm), iodine and Dragondorff reagent staining.

### 1.3 Spectroscopy.

Each synthetic step was characterized by NMR and Mass. ¹H and ¹³C NMR spectra were recorded on a Bruker 250 MHz and 500 MHz spectrometer (¹H; internal reference CDCl₃ at 7.27 ppm or D₂O at 4.75 ppm; ¹³C; internal reference CDCl₃ at 77.0 ppm). All experiments were performed at 23°C.

Electrospray mass spectra (ESI-MS) were recorded on SL 1100 system (Agilent, Germany) with ion-trap detection in positive and negative ion mode. HRMS were measured on a Thermo Finnigan LQT.

### 1.4 NMR experiments.

All NMR experiments with metal chelators described herein were performed at the Department of Chemistry of Technische Universität München (TUM) on a Bruker AV500 500 MHz Nuclear Magnetic Resonance Spectrometer equipped with a cryoprobe. pH values were measured with a standard pH electrode.

All solutions were prepared in MOPS buffer (30 mM) and deuterated water in a ratio of 1:1, at pH 7.4 and were measured at the temperature of 300K. ¹³C-methanol was used as a ¹³C reference.

A thermal spectrum was acquired (number of scans (ns) 1000, number of points (np) 16) using a 30° pulse and 90 degree pulse for decoupling. ¹³C T₁ values were calculated from a series of spectra acquired with varying repetition times (from 0.01 s to 50 s) by fitting the peak for each experiment to a classical NMR longitudinal relaxation theory. The assignment of the NMR-peaks of all compounds was done using NMR prediction software (ChemSketch - ACDLABS 12.0) and standard 1D and 2D-NMR-spectroscopy.

### 1.5 Synthesis and characterization of chemical shift metal sensors.

### (1) ¹³C - labeled EGTA

### ([3,12- bis(carboxymethyl)-6,9-dioxa-3,12-diazatetradecane-1,14-dioic acid)].

Labelled ¹³C -Labeled EGTA was synthesized in two steps as described in the literature (Ellis-Davies *et al.*). A solution of 2,2'-(ethane-1,2-diylbis(oxy))diethanamine (0.1 g, 0.68 mmol) and K₂CO₃ (0.56 g, 4.06 mmol) in anhydrous MeCN was stirred at room temperature for 30 min. ¹³C - labeled ethyl bromoacetate-1¹³C (0.34 mL, 3.04 mmol) was added dropwise and the reaction mixture was heated at 80°C for 4 h. The reaction progress was monitored by TLC. Upon consumption of starting materials, the solvent was filtered through G-4 cintered funnel and solvent was removed under reduced pressure. The crude residue was purified by column chromatography (100 % DCM to 95:5 DCM/MeOH; R_{f}=0.4) to give ¹³C - labeled *dimethyl 3,12-bis(2-methoxy-2-oxoethyl)-6, 9-dioxa-3,12-diazatetradecane-1,14-dioate* (0.295 g, 88%) as a yellow gum.

¹H NMR (CDCl₃, 250 MHz) δ ppm: 1.24 (t, *J*=6 Hz, 12H), 2.94 (t, *J*=6 Hz, 4H), 3.42 - 3.70 (m, 16H), 4.13 (q, J=7.0 Hz, 8H). ¹³C NMR (CDCl₃, 62.9 MHz) δ ppm: 14.1, 53.6, 55.3, 56.2, 60.4, 70.1, 171.4. HR-MS (ES⁺) *m*/*z* ¹²C₁₈¹³C₄H₄₁N₂O₁₀ requires 497.2895 [M+H]⁺; found 497.2880 [M+H]⁺.

The tetra-acid (*3,12-bis(carboxymethyl)-6,9-dioxa-3,12-diazatetradecane-1,14-dioic acid*) was obtained by selective deprotection of the ethyl group on ¹³C-labeled *dimethyl 3,12-bis(2-methoxy-2-oxoethyl)-6,9-dioxa-3,12-diazatetradecane-1,14-dioate* (0.25 g, 0.65 mmol) with NaOH (0.25 g, 3 mmol) in 10 mL MeOH: H₂O (9:1) for 2 h at room temperature. After reaction completion, the pH was adjusted to 7.4 by 3N HCl. The solvent was evaporated under reduced pressure to afford ¹³C-labeled EGTA (0.19 g, 100% w/w) as an off-white solid.

¹H NMR (D₂O, 250 MHz) δ ppm: 3.23 (t, *J*=7 Hz, 4H), 3.54 - 3.76 (m, 12H), 3.80 (t, *J*=7.0 Hz, 4H). ¹³C NMR (D₂O, 62.9 MHz) δ ppm: 54.5, 58.7, 65.9, 69.6, 170.4. HR-MS (ES⁺) *m*/*z* ¹²C₁₀¹³C₄H₂₅N₂O₁₀ requires 385.1643 [M+H]⁺; found 385.1634 [M+H]⁺.

### (2) ¹³C-labeled EDTA

### [2,2',2",2"'-(ethane-1,2-diylbis(azanetriyl))tetraacetic acid].

¹³C-Labeled EDTA was synthesized in two steps as for the **¹³C** -Labeled EGTA synthesis described above. A solution of ethane-1,2-diamine (0.1 g, 1.67 mmol) and K₂CO₃ (1.38 g, 10 mmol) in anhydrous MeCN was stirred at room temperature for 30 min. ¹³C - labeled ethyl bromoacetate-1¹³C (0.84 mL, 7.5 mmol) was added drop wise and the reaction mixture was heated at 80°C for 4 h. The reaction progress was monitored by TLC. Upon consumption of starting materials, the solvent was filtered through G-4 cintered funnel and solvent was removed under reduced pressure. The crude residue was purified by column chromatography (100 % DCM to 95:5 DCM/MeOH; R_{f}=0.5) to give ¹³C - labeled *tetraethyl 2,2',2",2'"'-(ethane-1,2-diylbis(azanetriyl))tetraacetate* (0.625 g, 92%) as a yellow gum. ¹H NMR (CDCl₃, 250 MHz) □ ppm: 1.28 (t, *J*=7.0 Hz, 12H), 2.92 (t, *J*=6.0 Hz, 4H), 3.61 (s, 8H), 4.17 (q, *J*=7.0 Hz, 8H). ¹³C NMR (CDCl₃, 62.9 MHz))□□ ppm: 14.1, 51.4, 55.3, 60.4, 171.6. HR-MS (ES⁺) *m*/*z* ¹²C₁₄¹³C₄H₃₃N₂O₈ requires 409.2371 [M+H]⁺; found 409.2365 [M+H]⁺.
The tetra-acid (*2,2',2",2'"-(ethane-1,2-diylbis(azanetriyl))tetraacetic acid)* was obtained by selective deprotection of the ethyl group on ¹³C - labeled *tetraethyl 2,2',2",2"'-(ethane-1,2-diylbis(azahetriyl))tetraacetate* (0.6 g, 2.05 mmol) with NaOH (0.36 g, 9 mmol) in 10 mL MeOH: H₂O (9:1) for 2 h at room temperature. After the reaction was completed, the pH was adjusted to 7.4 by 3N HCl. The solvent was evaporated under reduced pressure to afford ¹³C-labeled EDTA (0.435 g, 100% w/w) as an off-white solid.
¹H NMR (D₂O, 250 MHz) ) δ ppm: 3.71 (s, 4H), 3.94 (d, *J*=4.5, 8H). ¹³C NMR (D₂O, 62.9 MHz) δ ppm: 51.7, 58.5, 170.8. HR-MS (ES⁺) *m*/*z* ¹²C₆¹³C₄H₁₇N₂O₈ requires 297.1119 [M+H]⁺; found 297.1118 [M+H]⁺.

### (3) Bromo derivative of ¹³C-labeled Pyro-EGTA

### [2,2',2",21'"-(2,2'-(4-bromo-1,2-phenylene)bis(oxy)bis(ethane-2,1-diyl)) bis(azanetriyl)tetraacetic acid]

The bromo-derivative of ¹³C-labeled Pyro-EGTA was synthesized in 5 steps following a similar synthesis in the literature (Mishra *et al.,* 2011). The methyl groups were removed by using successive addition of borontribromide in the solution of *4-bromo-1,2-dimethoxybenzene* in anhydrous CH₂Cl₂ at -4°C to room temperature for 2 h. The reaction was quenched by MeOH and solvent was evaporated under reduced pressure to afford *4-bromobenzene-1,2-diol.* Stepwise alkylation of *4-bromobenzene-1,2-diol* with *tert*-butyl 2-bromoethylcarbamate in anhydrous MeCN (6 h at 80°C) afforded compound di-*tert*-butyl 2,2'-(4-bromo-1,2-phenylene)bis(oxy)bis(ethane-2,1-diyl)dicarbamate, which was de-Boc by using 4N HCl. Dioxane solution (30 min at room temperature) to give 2,2'-(4-bromo-1,2-phenylene)bis(oxy)diethanamine in good yield.
A solution of 2,2'-(4-bromo-1,2-phenylene)bis(oxy)diethanamine (0.1 g, 0.37 mmol) and K₂CO₃ (0.40 g, 2.91 mmol) in anhydrous MeCN was stirred at room temperature for 30 min. ¹³C - labeled ethyl bromoacetate-1¹³C (0.185 mL, 1.64 mmol) was added drop wise and the reaction mixture was heated at 80°C for 4 h. The reaction progress was monitored by TLC. Upon consumption of starting materials, the solvent was filtered through G-4 cintered funnel and solvent was removed under reduced pressure. The crude residue was purified by column chromatography (100 % DCM to 95:5 DCM/MeOH; R_{f}=0.55) to give ¹³C-labeled tetramethyl 2,2',2",2"'-(2,2'-(4-bromo-1,2-phenylene)bis(oxy)bis(ethane-2,1-diyl))bis(azanetriyl)tetraacetate (0.18 g, 82%) as a yellow gum.

¹H NMR (CDCl₃, 250 MHz) δ ppm: 1.25 (t, J=7.0, 12H), 3.05 - 3.33 (m, 4 H), 3.68 (d, J=5.0 Hz, 8H), 3.99 - 4.29 (m, 12H), 6.73 (d, J=9.0 Hz, 1H), 6.92 - 7.13 (m, 2H). ¹³C NMR (CDCl₃, 62.9 MHz) δ ppm: 14.2, 53.5, 55.5, 56.4, 60.5, 112.8, 117.2, 117.6, 123.7, 145.7. 147.3, 171.4. HR-MS (ES⁺) m/z ¹²C₂₂¹³C₄H₄₀BrN₂O₁₀ requires 623.2001 [M+H]⁺; found 623.1996 [M+H]⁺.

The tetra-acid [*2,2',2",2"'-(2,2'-(4-bromo-1,2-phenylene)bis(oxy)bis(ethane-2,1-diyl))bis(azanetriyl)tetraacetic acid*] was obtained by deprotection of the ethyl group on ¹³C-labeled tetramethyl 2,2',2",2'"-(2,2'-(4-bromo-1,2-phenylene)bis(oxy)bis(ethane-2,1-diyl))bis(azanetriyl)tetraacetate (0.15 g, 0.29 mmol) with NaOH (0.06 g, 1.46 mmol) in 10 mL MeOH: H₂O (9:1) for 2 h at room temperature. After reaction completion, the pH was adjusted to 7.4 by 3N HCl. The solvent was evaporated under reduced pressure to afford ¹³C-labeled *m*-Br-PyroEGTA (0.12 g, 100% w/w) as an off-white solid.

¹H NMR (D₂O, 250 MHz) ) δ ppm: 3.02 (t, J=5.0 Hz, 4H), 3.18 - 3.45 (m, 8H), 4.08 (t, J=5.0 Hz, 4H), 6.92 (d, J=8.50 Hz, 1H), 7.07 - 7.26 (m, 2H). ¹³C NMR (D₂O 62.9 MHz) δ ppm: 53.5, 58.3, 65.0, 112.4, 113.3, 115.2, 123.7, 146.6, 147.9, 179.4. HR-MS (ES⁺) *m*/*z* ¹²C₁₄¹³C₄H₂₄BrN₂O₁₀ requires 511.0749 [M+H]⁺; found 511.0746 [M+H]⁺.

### (4 and 5) APTRA [A= 2,2'-(2-(carboxymethoxy)phenylazanediyl)diacetic acid] and APTRA amide [B = 2,2'-(2-(2-morpholino-2-oxoethoxy)phenylazanediyl)diacetic acid].

Both compounds were synthesized in 4 steps as described in the literature (Otten *et al,* 2001, Dhingra *et al.*). Alkylation of the tert-butyl 2-hydroxyphenylcarbamate with methyl bromoacetate (for **A**)/ 2-bromo-1-morpholinoethanone (for **B**) in anhydrous MeCN (6 h at 80°C) gave the corresponding ester and amide of **A** [methyl 2-(2-(tert-butoxycarbonylamino)phenoxy)acetate] and **B** [tert-butyl 2-(2-morpholino-2-oxoethoxy)phenylcarbamate] respectively. These intermediates were further treated with mild acid (4N HCl Dioxane solution; (30 min at room temperature), cleaving the Boc group to afford the corresponding amines **A** [methyl 2-(2-aminophenoxy)acetate] and **B** [2-(2-aminophenoxy)-1-morpholinoethanone]. The ethyl di-esters of corresponding compounds **A** [diethyl 2,2'-(2-(2-methoxy-2-oxoethoxy)phenylazanediyl)diacetate] and **B** [diethyl 2,2'-(2-(2-morpholino-2-oxoethoxy)phenylazanediyl)diacetate] was obtained by alkylation with ethylbromoacetate in anhydrous DMF using proton sponge as strong base (48 h at 80°C). Following basic hydrolysis of these esters afforded resultant acids (**A** and **B**). After reaction completion, the pH was adjusted by 3N HCl. The solvent was evaporated under reduced pressure to afford (**A** and **B**) as dark yellow solids.

The features disclosed in the foregoing description, in the claims and/or in the accompanying drawings may, both separately and in any combination thereof, be material for realizing the invention in diverse forms thereof.

### REFERENCES

Angelovski, G., Chauvin, T., Pohmann, R., Logothetis, N. K., & Tóth, E. (2011). Calcium-responsive paramagnetic CEST agents. Bioorganic & Medicinal Chemistry, 19(3), 1097-1105. doi:10.1016/j.bmc.2010.07.023
Ardenkjaer-Larsen, J. H., Fridlund, B., Gram, A., Hansson, G., Hansson, L., Lerche, M. H., et al. (2003). Increase in signal-to-noise ratio of > 10,000 times in liquid-state NMR. Proceedings of the National Academy of Sciences of the United States of America, 100(18), 10158-10163.
Atanasijevic, T., Shusteff, M., Fam, P., & Jasanoff, A. (2006). Calcium-sensitive MRI contrast agents based on superparamagnetic iron oxide nanoparticles and calmodulin. Proceedings of the National Academy of Sciences of the United States of America, 103(40), 14707-14712. doi:10.1073/pnas.0606749103
Bar-Shir, A. et al. Metal Ion Sensing Using Ion Chemical Exchange Saturation Transfer (19)F Magnetic Resonance Imaging (2013). J Am Chem Soc. doi:10.1021/ja403542g
Amnon Bar-Shir; Nirbhay Yadav; Assaf A. Gilad; Peter C. van Zijl; Michael T. McMahon; Jeff W. Bulte. Poster, WMIC 2014, 19F probe allows simultaneous detection of multiple metal ions using iCEST MRI
Caravan, P. (2006). Strategies for increasing the sensitivity of gadolinium based MRI contrast agents. Chemical Society Reviews, 35(6), 512-523. doi:10.1039/b510982p
Carter, K. P., Young, A. M., & Palmer, A. E. (2014). Fluorescent Sensors for Measuring Metal Ions in Living Systems. Chemical Reviews, 114(8), 4564-4601. doi:10.1021/cr400546e
DeGraaf, Robin. In Vivo NMR Spectroscopy: Principles and Techniques, John Wiley & Sons; 2007.
Durham, A. C. H., & Walton, J. M. (1983). A survey of the available colorimetric indicators for Ca2+ and Mg2+ ions in biological experiments. Cell Calcium, 4(1), 47-55. doi: 10.1016/0143-4160(83)90048-9.
Ellis-Davies, G. C. R. (2008). Neurobiology with Caged Calcium. Chemical Reviews, 108(5), 1603-1613. doi:10.1021/cr078210i
Ernst, Richard. Principles of Nuclear Magnetic Resonance in One and Two Dimension (International Series of Monographs on Chemistry), Oxford University Press; 1997.
Henry, P.-G., Marjanska, M., Walls, J. D., Valette, J., Gruetter, R., & U urbil, K. (2006). Proton-observed carbon-edited NMR spectroscopy in strongly coupled second-order spin systems. Magnetic Resonance in Medicine : Official Journal of the Society of Magnetic Resonance in Medicine / Society of Magnetic Resonance in Medicine, 55(2), 250-257. doi:10.1002/mrm.20764
Jindal, A. K., Merritt, M. E., Suh, E. H., Malloy, C. R., Sherry, A. D., & Kovács, Z. (2010). Hyperpolarized 89Y complexes as pH sensitive NMR probes. Journal of the American Chemical Society, 132(6), 1784-1785. doi:10.1021/ja910278e
Looger, L. L. (2013). Genetically encoded calcium indicators for multi-color neural activity imaging and combination with optogenetics, 1-29. doi:10.3389/fnmol.2013.00002/abstract
Mishra, A., Logothetis, N. K., & Parker, D. (2011). Critical In Vitro Evaluation of Responsive MRI Contrast Agents for Calcium and Zinc. Chemistry - a European Journal, 17(5), 1529-1537. doi:10.1002/chem.201001548
Nelson SJ, Ozhinsky E, Li Y, Park I, Crane J. Strategies for rapid in vivo 1H and hyperpolarized 13C MR spectroscopic imaging. J Magn Reson 2013 ;229: 187-97.
Nelson SJ, Kurhanewicz J, Vigneron DB, Larson PE, Harzstark AL, Ferrone M, van Criekinge M, Chang JW, Bok R, Park I, Reed G, Carvajal L, Small EJ, Munster P, Weinberg VK, Ardenkjaer-Larsen JH, Chen AP, Hurd RE, Odegardstuen LI, Robb FJ, Tropp J, Murray JA. Metabolic Imaging of Patients with Prostate Cancer Using Hyperpolarized [1-13C]Pyruvate. Sci Trans Med. 2013 Aug 14;5(198): 198ra108.
Nonaka, H., Hata, R., Doura, T., Nishihara, T., Kumagai, K., Akakabe, M., et al. (2013). A platform for designing hyperpolarized magnetic resonance chemical probes. Nature Communications, 4, 1-7. doi:10.1038/ncomms3411
Ntziachristos, V. (2010). Going deeper than microscopy: the optical imaging frontier in biology. Nature Methods, 7(8), 603-614. doi:10.1038/nmeth.1483
Otten, P. A., London, R. E., & Levy, L. A. (2001). A new approach to the synthesis of APTRA indicators. Bioconjugate Chemistry, 12(1), 76-83.
Perez, J. M., Josephson, L., O'Loughlin, T., Hogemann, D., & Weissleder, R. (2002). Magnetic relaxation switches capable of sensing molecular interactions. Nature Biotechnology, 20(8), 816-820. doi:10.1038/nbt720
Que, E. L., & Chang, C. J. (2010). Responsive magnetic resonance imaging contrast agents as chemical sensors for metals in biology and medicine. Chemical Society Reviews, 39(1), 51-60. doi:10.1039/B914348N
V. Rieke, K. Vigen, G. Sommer, B. Daniel, J. Pauly, K. Butts, Referenceless PRF Shift thermometry, MRM, 2004 Jun;51 (6): 1223-31.
Robitaille, P. M., & Jiang, Z. (1992). New calcium-sensitive ligand for nuclear magnetic resonance spectroscopy. Biochemistry, 31(50), 12585-12591. doi:10.1021/bi00165a007
F A Schanne, T. L. D. R. K. G. J. F. R. (1990). Development of 19F NMR for measurement of [Ca2+]i and [Pb2+]i in cultured osteoblastic bone cells. Environmental Health Perspectives, 84, 99*.*
Shapiro, M. G., Atanasijevic, T., Faas, H., Westmeyer, G. G., & Jasanoff, A. (2006). Dynamic imaging with MRI contrast agents: quantitative considerations. Magnetic Resonance Imaging, 24(4), 449-462. doi:10.1016/j.mri.2005.12.033
Smith, G. A., Hesketh, R. T., Metcalfe, J. C., Feeney, J., & Morris, P. G. (1983). Intracellular calcium measurements by 19F NMR of fluorine-labeled chelators. Proceedings of the National Academy of Sciences of the United States of America, 80(23), 7178-7182.
Steenbergen, C., Murphy, E., Levy, L., & London, R. E. (1987). Elevation in cytosolic free calcium concentration early in myocardial ischemia in perfused rat heart. Circulation Research, 60(5), 700-707. doi:10.1161/01.RES.60.5.700
Verma, K. D., Forgács, A., Uh, H., Beyerlein, M., Maier, M. E., Petoud, S., et al. (2013). New Calcium-Selective Smart Contrast Agents for Magnetic Resonance Imaging. Chemistry - a European Journal, 19(52), 18011-18026.
Viale, A. & Aime, S. (2010). Current concepts on hyperpolarized molecules in MRI. Current Opinion in Chemical Biology 14, 90-96.
Winer, J. L., Liu, C. Y., & Apuzzo, M. L. J. (2012). The use of nanoparticles as contrast media in neuroimaging: a statement on toxicity. World Neurosurgery, 78(6), 709-711. doi:10.1016/j.wneu.2011.08.013

## Claims

1. Use of a compound with at least one metal-sensitive chemical shift for determining metal concentrations and/or measuring metal concentration changes,
wherein the compound is selected from the group of:
pyro-EGTA,
EGTA,
EDTA,
AATA,
APTRA,
BAPTA,
HIDA,
citrate,
carboxyglutamate (CGlu),
or derivatives thereof;
arylazo chromotopic acid derivatives,
beta-diketone (crown-ether) derivatives,
mono-, di-, or tri-pyridyl aniline derivatives,
mono-, di-, or tri-pyridyl amine derivatives,
trimethylphenylammonium derivatives.
and their hydrates, salts, solutions, stereoisomers.

2. The use of claim 1, wherein the compound is ¹H-labelled, ¹³C-labelled, ¹⁵N-labelled, ¹⁹F-labelled, ²⁹Si-labelled, ³¹P-labelled, and/or ⁸⁹Y-labelled and preferably exhibits at least one metal-sensitive ¹H and/or ¹³C and/or ¹⁵N and/or ¹⁹F and/or ²⁹Si and/or ³¹P and/or ⁸⁹Y chemical shift,
preferably a chemical shift that is specific for the particular metal,
and/or wherein the compound furthermore exhibits at least one metal-insensitive chemical shift, preferably one metal-insensitive ¹H and/or ¹³C and/or ¹⁵N and/or ¹⁹F and/or ²⁹Si and/or ³¹P and/or ⁸⁹Y chemical shift, preferably a shift with a narrow bandwidth.

3. The use of claim 1 and 2, wherein a derivative or analog of pyro-EGTA, EGTA, EDTA, AATA, APTRA, BAPTA, HIDA, citrate, carboxyglutamate (CGlu), arylazo chromotopic acid, beta-diketone (crown-ether), mono-, di-, or tri-pyridyl aniline, mono-, di-, or tri-pyridyl amine, trimethylphenylammonium is selected from
acids, amides, esters, ethers, pyridine, ketone,
fluorinated analogs,
including perdeuterated analogs, ¹⁸F radioisotopes.
and their hydrates, salts, solutions, stereoisomers,
and/or compounds comprising at least one carboxylate, amide, nitrogen, oxygen group involved in coordinating of metals.

4. The use of any of claims 1 to 3, wherein the compound comprises further component(s),
preferably covalently attached via functional or anchoring group(s),
wherein the further component(s) is/are selected from:
- label(s)
such as fluorophore(s), chromophore(s), NIR dye(s),
(e.g. cyanines for example indocyanine green (ICG) and derivatives, bodipy derivatives, squarine derivatives, IR780 and derivatives and fluorescent proteins and chromoproteins)
radioisotope(s);
- photo- and/or thermolabile moiety/moieties
such as 6-nitroveratroyloxycarbonyl group (NVOC),
- macrocycle
preferably to stably hold a metal
- nanostructure(s),
including magnetic nanoparticles and ultrasound contrast agents,
and/or
- radiocontrast agent(s).

5. The use of any one of claims 1 to 4, wherein the compound is hyperpolarized, preferably by dynamic nuclear polarization (DNP),
and/or the ¹H nuclei and/or ¹³C nuclei and/or ¹⁵N nuclei and/or ¹⁹F nuclei and/or ²⁹Si nuclei and/or ³¹P nuclei and/or ⁸⁹Y nuclei belonging to the metal-sensitive and metal-specific chemical shift(s) of the compound exhibit(s) a long longitudinal relaxation time T₁ and/or an optimized dynamic range of the metal concentration of interest.

6. A biosensor for determining metal concentrations and/or measuring metal concentration changes,
comprising
(i) at least one compound with at least one metal-sensitive chemical shift as defined in any one of claims 1 to 5,
(ii) optionally, a reference compound,
(iii) optionally, one or several pharmaceutically acceptable carriers and/or excipients,
wherein, preferably, the reference compound is a compound which does not exhibit metal-sensitive chemical shift(s) in an NMR spectrum, preferably the reference compound is ¹H and/or ¹³C and/or ¹⁵N and/or ¹⁹F and/or ²⁹Si and/or ³¹P and/or ⁸⁹Y-labelled,
and preferably exhibits at least one metal-insensitive ¹H and/or ¹³C and/or ¹⁵N and/or ¹⁹F and/or ²⁹Si and/or ³¹P and/or ⁸⁹Y chemical shift.

7. The compound of any of claims 1 to 5 or the biosensor of claim 6 for use in *in vivo* magnetic resonance imaging (MRI), magnetic resonance spectroscopy (MRS) or magnetic resonance tomography (MRT)
and/or absorbance/transmission, reflection, fluorescence or optoacoustic measurements and imaging.

8. The compound of any of claims 1 to 5 or the biosensor of claim 6 for use in diagnosing and/or monitoring treatment of diseases causing changes in metal concentrations, wherein a disease causing changes in metal concentrations is preferably selected from:
- diseases in which calcium signaling is affected
as in:
neuropsychiatric diseases, such as epilepsy, stroke,
brain damage,
neurodegenerative diseases,
states of altered neuronal processing, such as sleep, coma, anesthesia, intoxication,
cardiovascular diseases, such as arrhythmias, cardiac ischemia and myocardial infarct,
neuromuscular or muscular diseases,
- diseases in which calcium and/or magnesium uptake, storage, utilization or excretion is affected
as in
endocrinological conditions, such as hyper/hypoparathyroidism,
malnutrition and gastrointestinal diseases, such as malabsorption and diarrhea e.g. due to alcoholism,
bone-related diseases, such as osteoporosis,
kidney diseases and treatment with diuretics,
osteoclastic processes of tumors or infections,
diseases in which calcification in tissues occurs, such as cancers, including breast cancer,
atherosclerotic alterations or inflammatory processes,
due to other medical treatments,
- diseases in which iron uptake, storage, utilization or excretion is affected as in
iron-deficit caused by *e.g.* malnutrition or blood loss, or anemia including genetically-caused anemias, infections and inflammation, neurodegenerative diseases, medical treatments
- diseases in which uptake, storage, utilization or excretion of other metals is affected as in
neurodegenerative diseases (*e.g.* zinc, copper), or
metal storage diseases, such as Wilson's disease, tumors, such as melanomas,
medical treatments,
- diseases caused by metal intoxications
with *e.g.* manganese, nickel, cobalt, lead, mercury.

9. The compound or biosensor of claim 8, wherein the imaging is real-time,
preferably comprising the resolution of the spatiotemporal metal distribution,
more preferably comprising the use of frequency encoding techniques, such as chemical shift imaging (CSI) and phase sensitive encodings of chemical shifts,
preferably comprising monitoring extra- and intracellular calcium signaling and (treatments of) metal metabolism and intoxication in preclinical animal models, such as zebrafish, rodents and non-human primates.

10. Use of the compound of any of claims 1 to 5 as metal sensor for *in vitro* or *ex-vivo* NMR spectroscopy,
preferably comprising monitoring extra- and intracellular calcium signaling and (treatments of) metal metabolism and toxicity in tissue culture and cell lines.

11. An *in vitro* method for determining metal concentration and/or measuring metal concentration changes,
comprising the steps of
(i) providing a sample,
(ii) adding a compound with at least one metal-sensitive chemical shift of any of claims 1 to 5 or a biosensor of claim 6 to the sample,
(iii) performing magnetic resonance imaging (MRI) or magnetic resonance spectroscopy (MRS) and thereby determining the spatial distribution of metal concentration or metal concentration changes of or in the sample
(1) by obtaining a chemical shift difference between at least one metal-sensitive chemical shift of the compound and a metal-independent chemical shift, such metal-independent chemical shift acting as a reference chemical shift, or
(2) by measurement of the absolute chemical shift, or
(3) by measuring chemical shift differences involving at least one metal-sensitive shift, or
(4) by obtaining additional measurements of metal-insensitive signals that relate to the concentration of the sensor, for instance based on radioactivity, absorbance, fluorescence or optoacoustic signal from the metal-sensor containing the appropriate radioactive moiety, fluorophore/chromophore,
preferably simultaneously measuring the spatiotemporal distribution of several metals at once based on the metal-specific chemical shifts,
wherein the sample is preferably a cell culture sample (such as derived from a human or non-human body, ex vivo tissue, cell culture),
and/or wherein step (iii) is preferably carried out in an MRI scanner machine with MRS or MRSI capabilities or in a NMR spectrometer.

12. An *in vivo* method for determining metal and/or measuring metal concentration changes, preferably in real-time,
comprising the steps of
(i) applying or administering a compound with at least one metal-sensitive chemical shift of any of claims 1 to 5 or a biosensor of claim 6 to the body of a patient or non-human animal,
(ii) performing magnetic resonance imaging (MRI) and thereby determining one or several metal concentrations or metal concentration changes of or in the body of said patient or non-human animal
(1) by correcting a spatiotemporally resolved metal-sensitive signal for differences in local and time-dependent signal intensities, e.g. due to differences in the local concentration of the metal sensors, and
by obtaining the chemical shift and/or its integrated peak area and/or peak amplitude from at least one metal-sensitive chemical shift and a metal-insensitive reference in the form chemical shift and/or integrated peak area from at least one metal-insensitive chemical shift or a function of metal-sensitive shifts,
or
(2) by measurement of the absolute chemical shift, integrated peak area or peak amplitude,
or
(3) by measuring a metal-insensitive MRI signal from another nucleus such as proton via a lanthanide, ¹H and/or ¹³C and/or ¹⁵N and/or ¹⁹F and/or ²⁹Si and/or ³¹P and/or ⁸⁹Y, or
(4) by combination with a PET measurement *e.g.* via ¹⁸F or fluorescence or optoacoustics *e.g.* via a chromophore or fluorophore,
wherein the patient can preferably be diagnosed with a disease causing changes in metal concentrations or the treatment of a disease causing changes in metal concentrations can be monitored,
wherein a disease causing changes in metal concentrations is preferably selected from the diseases as defined in claim 8.

13. The method of claim 11 or 12, wherein the metal-independent chemical shift (reference chemical shift) is from the same compound, *i.e.* the compound with at least one metal-sensitive chemical shift, or from another substance, and is used as a metal-independent reference.

14. A method of diagnosing and/or monitoring treatment of a disease causing changes in metal concentrations,
comprising the steps of
(i) applying or administering a compound with at least one metal-sensitive chemical shift of any of claims 1 to 5 or a biosensor of claim 6 to the body of a patient or non-human animal,
(ii) performing magnetic resonance imaging (MRI) or magnetic resonance spectroscopy (MRS) and thereby determining one or several metal concentrations or metal concentration changes of or in the body of said patient or non-human animal
(1) by obtaining over time the chemical shift and/or its integrated peak area and/or its peak amplitude from at least one metal-sensitive chemical shift and a metal-insensitive reference in the form of a chemical shift and/or its integrated peak area and/or peak amplitude from at least one metal-insensitive chemical shift or a function of metal-sensitive shifts,
or
(2) by measurement of the absolute chemical shift, integrated peak area or peak amplitude,
or
(3) by measuring a metal-insensitive MRI signal from another nucleus such as proton via a lanthanide, ¹H and/or ¹³C and/or ¹⁵N and/or ¹⁹F and/or ²⁹Si and/or ³¹P and/or ⁸⁹Y, or
(4) by combination with a PET measurement *e.g.* via ¹⁸F or fluorescence or optoacoustics e.g. via a chromophore or fluorophore, or CT via a radiocontrast agent, (iii) calculating metal concentration maps based on spatially resolved metal concentration values or metal concentration changes determined in step (ii),
wherein a disease causing changes in metal concentrations is preferably selected from the diseases as defined in claim 8,
wherein step (iii) preferably comprises
comparing said relative chemical shifts to predetermined or simultaneously measured calibration curves of the compound with at least one metal-sensitive chemical shift in solutions with known metal concentration or mixtures of metals with known concentrations,
and/or said method furthermore comprises
hyperpolarizing the compound with at least one metal-sensitive chemical shift before application or administration to the body of the patient.

15. The method of claim 14, comprising magnetic resonance spectroscopy (MRS) or magnetic resonance tomography (MRT),
and/or wherein the imaging is real-time.

16. The method of any one of claims 11 to 15, comprising the resolution of a spatial metal concentration distribution,
preferably comprising the use of frequency encoding techniques, such as comprising chemical shift imaging (CSI) and phase sensitive encodings of chemical shifts as well as indirect methods based on detecting protons bound to the heteronucleus, such as ¹³C, *e.g.* proton-observed carbon-edited (POCE) sequences and its analogs, as well as CEST or Magnetization Transfer (MT) methods.

17. Use of the compound of any of claims 1 to 5 or the biosensor of claim 6 in quality control of food or in the examination of plants and organisms,
or for monitoring of environmental resources including air, water and soil with respect to pollution and harmful metal concentrations.

18. A derivative or analog of pyro-EGTA, AATA, APTRA or BAPTA, comprising metal affinity modifying moiety/moieties,
wherein the metal affinity modifying moiety/moieties is/are selected from halogen (*e.g.* Cl, Br, I), a hyperpolarizable nucleus (e.g. ¹⁹F, or ³¹P, ²⁹Si, ¹⁵N) or NH₂,
such as Bromo-pyro-EGTA, ¹⁹F-pyro-EGTA.

19. The derivative or analog of pyro-EGTA, AATA, APTRA or BAPTA of claim 18 for use according to any of the claims 1 to 17.
